(19) Europäisches Patentamt
European Patent Office
Office européen des brevets

(11) Veröffentlichungsnummer: **0 186 010 B1**

(12) **EUROPÄISCHE PATENTSCHRIFT**

(45) Veröffentlichungstag der Patentschrift: **31.01.90**

(21) Anmeldenummer: **85115547.3**

(22) Anmeldetag: **06.12.85**

(51) Int. Cl.⁴: **C 07 D 487/04,** C 07 D 487/10, A 61 K 31/415 // C07D209/34, C07D209/42 ,(C07D487/04, 235:00, 209:00)

# (54) Neue Pyrrolo-Benzimidazole, Verfahren zu ihrer Herstellung und diese Verbindungen enthaltende Arzneimittel.

(30) Priorität: **14.12.84 DE 3445669**

(43) Veröffentlichungstag der Anmeldung: **02.07.86 Patentblatt 86/27**

(45) Bekanntmachung des Hinweises auf die Patenterteilung: **31.01.90 Patentblatt 90/5**

(84) Benannte Vertragsstaaten: **AT BE CH DE FR GB IT LI LU NL SE**

(56) Entgegenhaltungen:
**EP-A- 0 098 448**
**EP-A- 0 161 632**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31 (DE)**

(72) Erfinder: **Mertens, Alfred, Dr., In der Schanz 31, D-6905 Schriesheim (DE)**
Erfinder: **Hölck, Jens-Peter, Dr. rer. nat., Ph. Brunnemer Weg 33, D-6800 Mannheim 31 (DE)**
Erfinder: **Kampe, Wolfgang, Dr. rer. nat., Zedernstrasse 49, D-6805 Heddesheim (DE)**
Erfinder: **Müller-Beckmann, Bernd, Dr., Hochgewanne 46, D-6718 Grünstadt (DE)**
Erfinder: **Strein, Klaus, Prof., Eichenstrasse 45, D-6944 Hemsbach (DE)**
Erfinder: **Schaumann, Wolfgang, Prof., Mönchhofstrasse 58, D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue Pyrrolo-Benzimidazole der allgemeinen Formel I

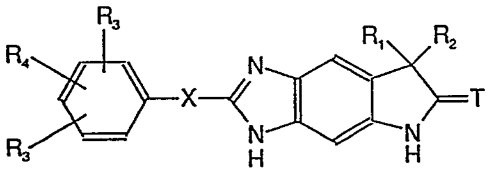

(I),

in welcher

$R_1$ ein Wasserstoffatom, eine $C_1$–$C_6$-Alkyl-, $C_2$–$C_6$-Alkenyl- oder eine $C_3$–$C_7$-Cycloalkylgruppe bedeutet,

$R_2$ ein Wasserstoffatom, eine $C_1$–$C_6$-Alkyl-, $C_2$–$C_6$-Alkenyl- oder Cyangruppe, eine durch eine Hydroxy-, $C_1$–$C_6$-Alkyl-, $C_1$–$C_6$-Alkoxy-, Amino-, $C_1$–$C_6$-Alkylamino-, Di-$C_1$–$C_6$-Alkylamino- oder Hydrazinogruppe substituierte Carbonylgruppe, oder mit $R_1$ zusammen eine $C_3$–$C_7$-Cycloalkylengruppe darstellt oder $R_1$ und $R_2$ zusammen eine $C_3$–$C_7$-Alkyliden- oder $C_3$–$C_7$-Cycloalkylidengruppe bilden,

$R_3$, $R_4$ und $R_5$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino- oder Dialkylamino- substituierte Sulfonylgruppe, eine Morpholino-, Pyrrolidino-, Piperidino- oder Hexamethyleniminosulfonylgruppe, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Trifluormethyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl- oder Cyanogruppe sein können, wobei die Alkylteile der oben genannten Substituenten 1-5 Kohlenstoffatome enthalten, und

X ein Valenzstrich, eine $C_1$-$C_4$-Alkylengruppe oder die Vinylengruppe sein kann,

T ein Sauerstoffatom darstellt, deren Tautomere und deren physiologisch verträgliche Salze anorganischer und organischer Säuren und Verfahren zu ihrer Herstellung, sowie diese Verbindungen enthaltende Arzneimittel.

Da die Verbindungen der allgemeinen Formel I für den Fall, dass $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen. Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbessern die Mikrozirkulation.

Aus EP-A 161 632 sind bereits Pyrrolo-Benzimidazol-Derivate bekannt, die sich von den erfindungsgemässen Verbindungen allerdings dadurch unterscheiden, dass sich in 2-Stellung des eingangs definierten Pyrrolo-Benzimidazol-Ringsystems anstelle einer Phenylgruppe eine Pyridylgruppe befindet.

In der allgemeinen Formel I kann der Substituent $R_1$ Wasserstoff, eine Alkyl-, Cycloalkyl- oder Alkenylgruppe sein und $R_2$ ein Wasserstoffatom, eine Alkyl- oder Alkenylgruppe, eine Cyangruppe oder eine durch eine Hydroxy-, Alkyl-, Alkoxy-, Amino-, Alkylamino-, Dialkylamino- oder Hydrazinogruppe substituierte Carbonylgruppe darstellen, wobei jeder der vorgenannten Alkyl- und Alkenylteile geradkettig oder verzweigt sein kann und 1-6 bzw. 2-6 Kohlenstoffatome und der vorgenannte Cycloalkylteil 3-7 Kohlenstoffatome enthalten kann.

Bevorzugt in diesem Sinne ist für $R_1$ Wasserstoff, die Methyl-, Ethyl-, Isopropyl-, 3-Pentyl-, Allyl-, Cyclopentyl- oder Cyclohexylgruppe und $R_2$ kann vorzugsweise Wasserstoff, eine Methyl-, Ethyl-, Isopropyl-, 3-Pentyl-, Allyl-, Cyan-, Carboxy-, Acetyl-, Propionyl-, Methoxycarbonyl-, Ethoxycarbonyl-, Aminocarbonyl-, Methylaminocarbonyl-, Dimethylaminocarbonyl- und Hydrazinocarbonylgruppe darstellen.

$R_1$ und $R_2$ können zusammen mit dem C-Atom, an das sie gebunden sind, auch einen Cycloalkylring mit 3-7 Kohlenstoffatomen bilden, vorzugsweise handelt es sich dabei um die Spirocyclopropyl-, Spirocyclobutyl-, Spirocyclopentyl- und Spirocyclohexylgruppe.

$R_1$ und $R_2$ können zusammen auch eine $C_3$–$C_7$ Alkyliden- oder $C_3$–$C_7$ Cycloalkylidengruppe bilden, vorzugsweise handelt es sich dabei um die Isopropyliden- oder Cyclohexylidengruppe.

Der Alkylteil der bei $R_3$, $R_4$ und $R_5$ genannten Substituenten kann 1-5 Kohlenstoffatome, vorzugsweise 1-4 Kohlenstoffatome enthalten. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsufinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethylethansulfonylamino-, N-Isopropyl-ethansulfonylamino-, N-Methyl-n-propansulfonylamino-, N-n-Propyl-n-propansulfonylamino-, N-Methyltrifluormethansulfonylamino-, N-Ethyl-trifluromethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Methylaminosulfonyl-, Ethylaminosulfonyl-, n-Propylaminosulfonyl-, n-Butylaminosulfonyl-,

n-Pentylaminosulfonyl-, Dimethylaminosulfonyl-, N-Methyl-isopropylaminosulfonyl-, Acetylamino-, Propionylamino-, Methylaminocarbonylamino-, Ethylaminocarbonylamino-, Propylaminocarbonylaminogruppe, eine Methyl-, Ethyl-, Propyl-, Trifluormethyl-, Methoxy-, Ethoxy-, Propyloxygruppe, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxygruppe, eine Methylmercapto-, Ethylmercaptogruppe, eine Methylsulfinyl-, Ethylsulfinylgruppe, eine Methylsulfonyl- und Ethylsulfonylgruppe.

Sulfonylgruppen, die durch cyclische Iminogruppen substituiert sein können, sind bevorzugt die Morpholino-, Pyrrolidino-, Piperidino- und Hexamethyleniminosulfonylgruppen.

Insbesondere sind bevorzugt für

$R_3$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1–4 Kohlenstoffatomen, eine Alkylcarbonylamino-, Aminocarbonylamino- oder N-alkyl-aminocarbonylaminogruppe, eine Alkylmercapto-, Alkylsulfinyl- oder Alkylsulfonylgruppe, wobei jeder der vorgennanten Alkylteile 1 oder 2 Kohlenstoffatome enthalten kann, eine Halogen-, Amino-, Hydroxy-, Dialkylamino-, Alkyl-, Trifluormethyl-, Alkoxy-, Alkenyloxy- oder Alkinyloxygruppe vorzugsweise mit 1–3 Kohlenstoffatomen, eine Cyanmethyloxy-, Methoxycarbonylmethyloxy- oder die 1-Imidazolylgruppe,

für $R_4$ Wasserstoff, eine Alkylgruppe mit 1–3 Kohlenstoffatomen, eine Alkoxy- oder Dialkylaminogruppe mit 1 oder 2 Kohlenstoffatomen in jedem Alkylteil oder ein Halogenatom und

für $R_5$ Wasserstoff oder die Methoxygruppe.

Der Phenylteil kann 1 bis 3 der genannten Substituenten tragen.

Bevorzugte monosubstituierte Verbindungen sind die Hydroxy-, $C_1$–$C_3$ Alkyl-, Trifluormethyl-, $C_1$–$C_3$ Alkoxy-, Allyloxy-, Propargyloxy-, Cyanmethyloxy-, Methoxycarbonylmethyloxy-, Halogen- Nitro-, Amino-, $C_1$–$C_3$ Dialkylamino-, $C_1$–$C_3$ Alkylmercapto-, $C_1$–$C_3$ Alkylsulfinyl-, $C_1$–$C_3$ Alkylsulfonyl-, $C_1$–$C_3$ Alkylsulfonyloxy- und die 1-Imidazoylverbindungen, wobei der Substituent in 2-, 3- oder 4-Stellung stehen kann.

Bevorzugte disubstituierte Verbindungen enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Trifluormethyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substitutenten gleich oder verschieden sein können und in 2,3-, 2,4-, 2,5-, 2,6-, 3,4- und 3,5-Stellung bevorzugt jedoch in 2,4-, 2,5- und 3,4-Stellung stehen können und die vorgenannten Alkylreste, allein oder in Kombination mit anderen Resten, 1–3 C-Atome aufweisen können.

Bevorzugter trisubstituierter Phenylrest ist 3,4,5-Trimethoxyphenyl.

Für X ist der Valenzstrich, eine Methylen-, Ethylen- oder die Vinylengruppe bevorzugt.

T ist ein Sauerstoffatom.

Besonders bevorzugte Verbindungen sind Verbindungen der allgemeinen Formel I

in der

$R_1$ ein Wasserstoffatom, die Methyl- oder Ethylgruppe darstellt,

und $R_2$ die Methyl-, Ethyl-, Isopropyl-, Cyan-, Acetyl-, Methoxy-carbonyl-, Ethoxycarbonyl-, Aminocarbonyl- und Hydrazinocarbonylgruppe bedeuten,

$R_1$ und $R_2$ einen Spirocyclopentylring darstellen, wenn $R_1$ und $R_2$ mit dem C-Atom, an das sie gebunden sind, einen Cycloalkylring bilden,

$R_3$ ein Wasserstoff, die Methansulfonyloxy-, Trifluormethansulfonyloxy-, Methansulfonylamino-, Trifluormethansulfonylamino-, Methansulfonylmethylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino- oder die 1-Imidazolylgruppe darstellt,

$R_4$ Wasserstoff, die Methyl-, Methoxy-, Dimethylamino- oder Chlorgruppe bedeutet,

$R_5$ Wasserstoff oder die Methoxygruppe ist,

X ein Valenzstrich, eine Alkylengruppe mit 1 oder 2 Kohlenstoffatomen oder die Vinylengruppe ist und T für ein Sauerstoffatom steht.

Die Herstellung der Verbindungen der allgemeinen Formel I erfolgt zunächst in der Weise, dass man entweder

a) Verbindungen der allgemeinen Formel II

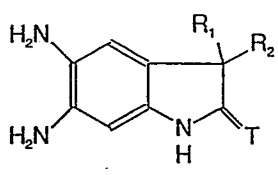

(II),

in der
$R_1$, $R_2$ und T die oben angegebene Bedeutung haben, mit einer Verbindung der allgemeinen Formel III

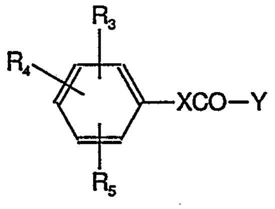

(III),

in der
$R_3$, $R_4$, $R_5$ und X die angegebene Bedeutung besitzen und Y entweder Wasserstoff oder ein leicht abspaltbarer Rest darstellt, umsetzt und die erhaltenen Verbindungen zu einer Verbindung der Formel I oder dessen tautomeren Form cyclisiert, und eine erfindungsgemäss erhaltene Verbindung der Formel I gewünschtenfalls in eine andere Verbindung der Formel I oder dessen Tautomer umwandelt und/oder eine erhaltene Verbindung der allgemeinen Formel I und dessen Tautomer in ein physiologisch verträgliches Säureadditionssalz mit einer anorganischen oder organischen Säure überführt.

Verbindungen der Formel II sind Gegenstand der deutschen Patentanmeldung P 3 417 643.8. Neue Verbindungen können nach den dort beschriebenen Verfahren hergestellt werden.

Unter Verbindungen der Formel III versteht man insbesondere Aldehyde, sowie Säurehalogenide wie Säurechloride, Carbonsäureester wie Methyl- und Ethylester und andere aktivierte Carbonsäurederivate, wie z.B. Anhydride.

Ist die Verbindung der Formel III ein Aldehyd findet die Umsetzung zur Schiff'schen Base mit Verbindungen der allgemeinen Formel II vorzugsweise in alkoholischem Medium statt, die anschliessende Cyclisierung und Oxidation zu den Verbindungen der allgemeinen Formel I erfolgt durch Erwärmen des Reaktionsansatzes zum Rückfluss in Gegenwart von Luftsauerstoff und katalytischen Mengen Säure, wie z.B. Toluolsulfonsäure.

Ist die Verbindung der allgemeinen Formel III ein Carbonsäurederivat, findet die Umsetzung mit Verbindungen der allgemeinen Formel II zum Amid in inerten Lösungsmitteln, vorzugsweise Methylenchlorid statt und die anschliessende Cyclisierung zu Verbindungen der allgemeinen Formel I wird in saurem Medium, vorzugsweise in Gegenwart von Mineralsäuren wie $H_2SO_4$ oder HCl in alkoholischer Lösung, vorgenommen.

b) Verbindungen der allgemeinen Formel IV

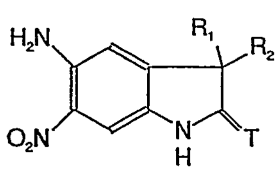

(IV),

oder V,

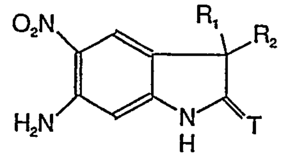

(V),

in denen
$R_1$, $R_2$ und T die angegebene Bedeutung haben, mit einer Verbindung der allgemeinen Formel III, in der
$R_3$, $R_4$, $R_5$ und X die angegebene Bedeutung besitzen und Y ein leicht abspaltbarer Rest darstellt, nach literaturbekannten Verfahren zu Verbindungen der allgemeinen Formel VI,

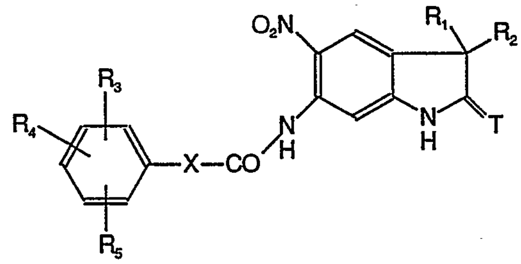

(VI),

oder VII,

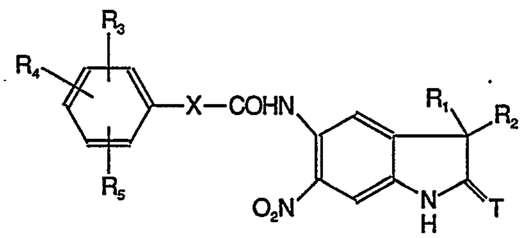

(VII)

in denen
$R_1$, $R_2$, $R_3$, $R_4$, $R_5$, X und T die angegebene Bedeutung haben, acyliert, und nach Hydrierung und Cyclisierung die gewünschten Verbindungen der Formel I erhält.

c) ausgehend von Verbindungen der Formel VIII

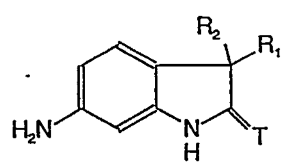

(VIII),

oder IX,

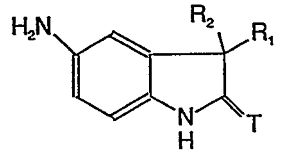

(IX),

in denen $R_1$, $R_2$ und T die oben angegebene Bedeutung besitzen, durch Umsetzung mit Verbindungen der allgemeinen Formel III, in denen Y einen leicht abspaltbaren Rest darstellt, nach allgemein bekannter Weise Verbindungen der Formel X

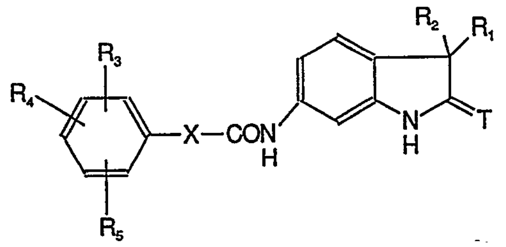

(X),

oder XI

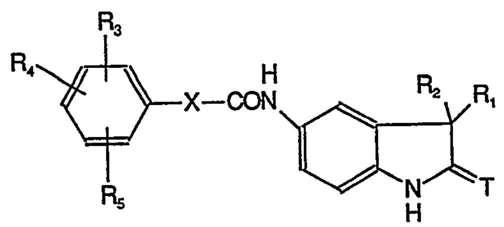

(XI),

in denen $R_1$, $R_2$, $R_3$, $R_4$, $R_5$, T und X die oben angegebenen Bedeutungen besitzen, erhält und in bekannter Weise durch Nitrierung in Verbindungen der allgemeinen Formel VI

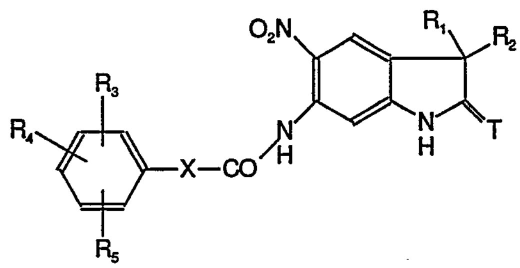

(VI),

oder VII,

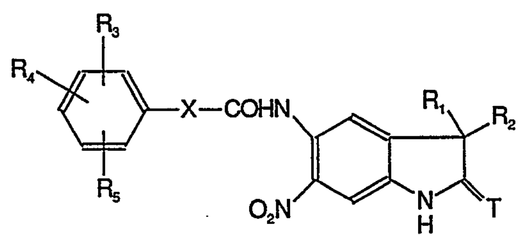

(VII),

in denen $R_1$, $R_2$, $R_3$, $R_4$, $R_5$, T und X die oben angegebenen Bedeutungen haben, überführt.

Nach Hydrierung der Nitrogruppe in den Verbindungen der allgemeinen Formel VI bzw. VII zu den entsprechenden Aminverbindungen, erhält man durch Cyclisierung die gewünschten Verbindungen der allgemeinen Formel I.

Verbindungen der Formel II, IV, V, VIII und IX sind Gegenstand der deutschen Patentanmeldung P 3 417 643.8. Neue Verbindungen können nach den dort beschriebenen Verfahren hergestellt werden.

Unter Verbindungen der Formel III versteht man insbesondere Säurehalogenide wie z.B. Säurechloride oder Carbonsäureester und andere aktivierte Carbonsäurederivate, wie z.B. Anhydride.

Die Umsetzung zu den Verbindungen der Formeln VI und VII wird vorzugsweise in inerten Lösungsmittel bei Temperaturen zwischen 0°C und dem Siedepunkt des Lösungsmittels gegebenenfalls unter Zusatz einer Hilfsbase wie z.B. Triethylamin oder Pyridin vorgenommen.

Die Hydrierung der Verbindungen der allgemeinen Formel VI und VII wird vorzugsweise in einem Lösungsmittel wie Wasser, Wasser/Ethanol, Methanol, Eisessig, Essigsäureethylester oder Dimethylformamid zweckmässigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen (II)sulfat, Zinn(II)chlorid, Natriumsulfid, Natriumhydrogensulfid oder Natriumdithionit, oder mit Hydrazin in Gegenwart von Raney-Nikkel bei Temperaturen zwischen 0 und 50°C, vorzugsweise jedoch bei Raumtemperatur, durchgeführt.

Die Cyclisierung der so erhaltenen 5-Amino- bzw. 6-Aminoindolin-2-on-Derivate wird zweckmässig in einem Lösungsmittel oder Lösungsmittelgemisch wie Ethanol, Isopropanol, Eisessig, Benzol, Chlorbenzol, Toluol, Xylol, Glycol, Glycolmonomethylether, Sulfolan, Dimethylformamid oder Tetralin beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliummethylat oder Kalium-tert.-butylat durchgeführt. Die Cyclisierung kann jedoch auch ohne Lösungsmittel und/oder Kondensationsmittel durchgeführt werden.

Eine Verbindung der Formel I, in der $R_1$ und $R_2$ gleich Wasserstoff bedeuten, lässt sich in eine andere Verbindung der Formel I nachträglich umwandeln. Dies betrifft z.B. auch die Umsetzung mit Verbindungen der allgemeinen Formel XII

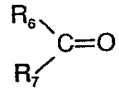

(XII),

in welcher

$R_6$ und $R_7$ Alkylgruppen sind oder $R_6$ zusammen mit $R_7$ eine $C_3$-$C_7$-Cycloalkylengruppe bildet, in Gegenwart einer Base wie Ammoniak oder Triethylamin in alkoholischer Lösung. Insbesondere trifft dieses zu für die Umwandlung von Verbindungen der allgemeinen Formel I in denen $R_1 = R_2$ = Wasserstoff bedeuten zu Verbindungen der allgemeinen Formel I in denen $R_1$ zusammen mit $R_2$ die Isopropylidengruppe, die Cyclopentyliden- oder Cyclohexylidengruppe darstellen, sowie gegebenenfalls deren Hydrierung zu den entsprechenden Verbindungen der allgemeinen Formel I, in der $R_1$ oder $R_2$ gleich Wasserstoff ist.

Die Herstellung von Verbindungen der allgemeinen Formel I, in der $R_3$ eine Alkylsulfinyl-, Al-

kylsulfonyl-, Alkylsulfinylmethyl- oder Alkylsulfonylmethylgruppe darstellt, wird durch nachträgliche Oxidation einer Verbindung der allgemeinen Formel XIII

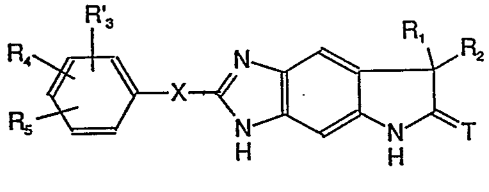
(XIII),

in der
$R_1$, $R_2$, $R_4$, $R_5$, X und T wie eingangs definiert sind und $R'_3$ eine Alkylmercapto- oder Alkylsulfenylmethylgruppe mit jeweils 13 Kohlenstoffatomen im Alkylteil ist, erreicht.

Die Oxidation wird vorzugsweise in einem Lösungsmittel oder Lösungsmittelgemisch, z. B. in Wasser, Wasser/Pyridin, Aceton, Eisessig, verdünnter Schwefelsäure oder Trifluoressigsäure, je nach dem verwendeten Oxidationsmittel zweckmässigerweise bei Temperaturen zwischen -80 und 100 °C durchgeführt.

Zur Herstellung einer Alkylsulfinyl- oder Alkylsulfinylmethylverbindung der allgemeinen Formel I wird die Oxidation zweckmässigerweise mit einem Äquivalent des verwendeten Oxidationsmittel durchgeführt, z. B. mit Wasserstoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenzoesäure in Methylenchlorid oder Chloroform bei -20 bis 60 °C, mit Natriummetaperjodat in wässrigem Methanol oder Ethanol bei 15 bis 25 °C, mit Brom in Eisessig oder wässriger Essigsäure, mit N-Brom-succinimid in Ethanol, mit tert.-Butyl-hypochlorid in Methanol bei -80 bis -30 °C, mit Jodbenzodichlorid in wässrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei -70 °C, der hierbei erhaltene Thioether-Chlor-Komplex wird zweckmässigerweise mit wässrigem Ethanol hydrolysiert.

Zur Herstellung einer Alkylsulfonyl- oder Alkylsulfonylmethylverbindung der allgemeinen Formel I wird die Oxidation zweckmässigerweise mit einem bzw. mit zwei oder mehr Äquivalenten des verwendeten Oxidationsmittels durchgeführt, z. B. Wasserstoffperoxid in Eisessig, Trifluoressigsäure oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

Die Herstellung von Verbindungen der allgemeinen Formel I, in der $R_3$ eine Alkansulfonyloxy-, Trifluormethansulfonyloxy-, Alkansulfonylamino-, N-alkyl-alkansulfonylamino-, trifluormethansulfonylamino-, oder N-Alkyl-trifluormethansulfonylaminogruppe darstellt, wird erreicht durch die nachträgliche Umsetzung einer Verbindung der allgemeinen Formel XIV

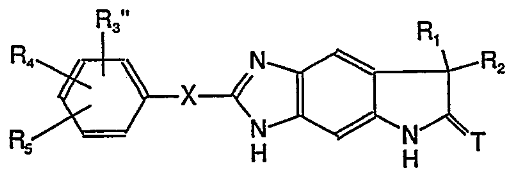
(XIV),

in der
$R_1$, $R_2$, $R_4$, $R_5$, X und T wie eingangs definiert sind und $R_3''$ eine Hydroxy-, Amino- oder N-Alkylaminogruppe mit 1-3 Kohlenstoffatomen im Alkylteil darstellt, mit einer Sulfonsäure der allgemeinen Formel XV

$R_8$-$SO_2OH$ (XV),

in der
$R_8$ eine Alyklgruppe mit 13 Kohlenstoffatomen oder eine Trifluormethylgruppe darstellt, in Gegenwart eines wasserentziehenden und/oder die Säure oder das Amin aktivierenden Mittels oder mit deren reaktionsfähigen Derivaten.

Die Umsetzung wird zweckmässigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel XV, z. B. mit deren Anhydrid oder Halogenid wie Methansulfonsäurechlorid oder Ethansulfonsäurechlorid, vorzugsweise bei Temperaturen zwischen 0 und 100 °C, z. B. bei Temperaturen zwischen Raumtemperatur und 50 °C, durchgeführt.

Die Herstellung von Verbindungen der allgemeinen Formel I, in der $R_2$ und/oder $R_3$ eine durch eine Amino-, Alkylamino-, Dialkylamino- oder Hydrazinogruppe substituierte Carbonyl- oder Sulfonylgruppe darstellt, wird erreicht durch die nachträgliche Umsetzung einer Verbindung der allgemeinen Formel XVI

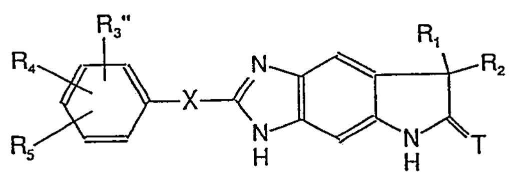
(XVI),

in der
$R_1$, $R_4$, $R_5$, X und T wie eingangs definiert sind und $R_2'$ und/oder $R_3'''$ eine Carboxyl- oder Hydroxy-

sulfonylgruppe darstellt, oder ein reaktionsfähiges Derivat hiervon wie z. B. Ester oder Säurechlorid mit Hydrazin oder einem Amin der allgemeinen Formel XVII

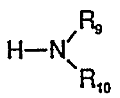

(XVII),

in der $R_9$ und $R_{10}$, die gleich oder verschieden sein können; Wasserstoffatome oder Alkylgruppen mit 1-5 Kohlenstoffatomen darstellen, oder mit einem reaktionsfähigen Derivat hiervon, falls $R_2'$ und/oder $R_3'''$ die Carboxyl- oder Hydroxysulfonylgruppe darstellt.

Die Umsetzung wird zweckmässigerweise in einem Lösungsmittel oder Lösungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z. B. in Gegenwart von Chlorameisensäureethylester, Thionylchlorid, Phosphortrichlorid, Phosphorpentoxid, N'N-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Hydrazino- oder Aminogruppe aktivierenden Mittels, z. B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, des weiteren kann während der Umsetzung entstehendes Wasser durch azeotrope Destillation, z. B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z. B. dem Carbonsäure- oder Sulfonsäurechlorid, und Hydrazin oder einem entsprechenden Amin, wobei diese gleichzeitig als Lösungsmittel dienen können, und bei Temperaturen zwischen 0 und 50°C durchgeführt.

Die Herstellung von Verbindungen der allgemeinen Formel I, in der $R_2$ eine Cyangruppe darstellt, kann durch nachträgliche Umsetzung einer Verbindung der allgemeinen Formel XVIII

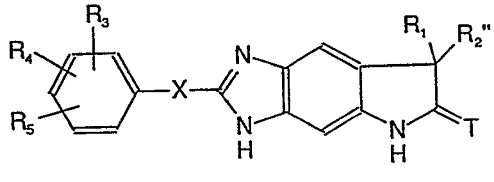

(XVIII),

erreicht werden,

in der $R_1$, $R_3$, $R_4$, $R_5$, X und T wie eingangs definiert sind und $R_2''$ eine Aminocarbonylgruppe darstellt.

Die Umsetzung wird zweckmässigerweise in einem inerten Lösungsmittel wie z. B. in Methylenchlorid, Chloroform, Dioxan, Pyridin, Xylol, Chlorbenzol in Gegenwart eines wasserentziehenden Mittels wie z. B. Thionylchlorid, Phosphortrichlorid, Phosphorpentoxid, Phosporpentachlorid, Aluminiumchlorid, Benzolsulfonsäurechlorid, Toluolsulfonsäurechlorid, Triphenylphosphin, Bortrifluorid oder Polyphosphorsäureethylester bei Temperaturen zwischen 50 und 250° vorzugsweise jedoch bei der Siedetemperatur des Lösungsmittels durchgeführt.

Erhält man eine Verbindung der allgemeinen Formel I, in der $R_2$ und/oder $R_3$ die Cyangruppe darstellt, so kann diese anschliessend mittels Alkoholyse und/oder Hydrolyse in eine entsprechende Verbindung, in der $R_2$ und/oder $R_3$ eine Alkoxycarbonylgruppe mit insgesamt 2-5 Kohlenstoffatomen, die Aminocarbonyl- oder Carboxylgruppe und/oder $R_4$ eine Alkoxycarbonylgruppe mit insgesamt 2-4 Kohlenstoffatomen, die Aminocarbonyl- oder Carboxylgruppe darstellen, überführt werden und/oder eine Verbindung der allgemeinen Formel I, in der $R_2$ und/oder $R_3$ die Carboxygruppe darstellt, mittels Veresterung in eine entsprechende Verbindung der allgemeinen Formel I, in der $R_2$ und/oder $R_3$ eine Alkoxycarbonylgruppe mit insgesamt 2-5 Kohlenstoffatomen darstellen, überführt werden.

Die nachträgliche Alkoholyse und/oder Hydrolyse wird zweckmässigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10 und 120°C, z. B. bei Temperaturen zwischen Raumtemperatur und der Siedetemperatur des Reaktionsgemisches, durchgeführt.

Die nachträgliche Veresterung wird zweckmässigerweise in einem geeigneten Lösungsmittel, z. B. in einem entsprechenden Alkohol, Pyridin, Toluol, Methylenchlorid, Tetrahydrofuran oder Dixoan, in Gegenwart eines säureaktivierenden und/oder wasserentziehenden Mittels wie Thionylchlorid, Chlorameisensäureethylester, N,N'-Carbonyldiimidazol oder N,N'-Dicyclohexylcarbodiimid oder dessen Isoharnstoffethern, gegebenenfalls in Gegenwart eines Reaktionsbeschleunigers wie Kupferchlorid, oder durch Umesterung, z. B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

Ferner können die erhaltenen Verbindungen der allgemeinen Formel I anschliessend gewünschtenfalls in ihre physiologisch verträglichen Säureadditionssalze mit anorganischen oder organischen Säuren überführt werden. Als

Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formel II bis V und VIII und IX sind teilweise literaturbekannt bzw. erhält man nach literaturbekannten Verfahren.

Wie bereits eingangs erwähnt weisen die neuen Verbindungen der allgemeinen Formel I, deren 1H Tautomere und deren physiologisch verträgliche Säureadditionssalze bei einer langen Wirkungsdauer überlegene pharmakologische Eigenschaften auf, insbesoondere eine blutdrucksenkende und/oder positiv-inotrope Wirkung und/oder beeinflussen sie die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die erfindungsgemässen neuen Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z. B. Tartrat und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z. B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süssstoffe enthalten.

Die erfindungsgemässen Verbindungen werden üblicherweise in Mengen von 10–500 mg pro Tag bezogen auf 75 kg Körpergewicht appliziert. Bevorzugt ist es, 2–3mal pro Tag 1–2 Tabletten mit einem Wirkstoffgehalt von 5–200 mg zu verabreichen. Die Tabletten können auch retardiert sein, wodurch nur noch 1mal pro Tag 1–2 Tabletten mit 10–500 mg Wirkstoff gegeben werden muss. Der Wirkstoff kann auch durch Injektion 1–8mal pro Tag bzw. durch Dauerinfusion gegeben werden, wobei Mengen von 5–200 mg/Tag normalerweise ausreichen.

Bevorzugt im Sinne der vorliegenden Erfindung sind ausser den in den Beispielen genannten Verbindungen die folgenden und deren Tautomere.

7,7-Dimethyl-2-(4-nitro-phenyl)-6,7-dihydro-3H,5H-pyrrolo[2,3-f]benzimidazol-6-on

7,7-Dimethyl-2-(4-amino-phenyl)-6,7-dihydro-3H,5H-pyrrolo[2,3-f]benzimidazol-6-on

7,7-Dimethyl-2-(2-hydroxy-phenyl)-6,7-dihydro-3H,5H-pyrrolo[2,3-f]benzimidazol-6-on

7,7-Dimethyl-(2-dimethylamino-4-nitro-phenyl)-6,7-dihydro-3H,5H-pyrrolo[2,3-f]benzimidazol-6-on

7,7-Dimethyl-2-(3,4-dichlor-phenyl)-6,7-dihydro-3H,5H-pyrrolo[2,3-f]benzimidazol-6-on

7,7-Dimethyl-2-(phenyl-vinyl)-6,7-dihydro-3H,5H-pyrrolo[2,3-f]benzimidazol-6-on, Mp. 320–325 °C

7,7-Dimethyl-2-(3,4-dimethoxy-phenyl-vinyl)-6,7-dihydro-3H,5H-pyrrolo[2,3-f]benzimidazol-6-on

7,7-Dimethyl-2-(2-methoxy-4-aminocarbonylamino-phenyl)-6,7-dihydro-3H,5H-pyrrolo[2,3-f]-benzimidazol-6-on

7,7-Dimethyl-2-(2-methoxy-4-methylaminocarbonylamino-phenyl)-6,7-dihydro-3H,5H-pyrrolo[2,3-f]benzimidazol-6-on

7,7-Dimethyl-2-(2-methoxy-4-trifluormethylsulfonyloxy-phenyl)-6,7-dihydro-3H,5H-pyrrolo-[2,3-f]benzimidazol-6-on

7,7-Dimethyl-2-(2-methoxy-4-ethylsulfonyloxy-phenyl)-6,7-dihydro-3H,5H-pyrrolo[2,3-f]benzimidazol-6-on

7,7-Dimethyl-2-(2-methoxy-4-aminosulfonyl-phenyl)-6,7-dihydro-3H,5H-pyrrolo[2,3-f]benzimidazol-6-on

7,7-Dimethyl-2-(2-methoxy-4-dimethylaminosulfonyl-phenyl)-6,7-dihydro-3H,5H-pyrrolo-[2,3-f]benzimidazol-6-on

7,7-Dimethyl-2-(2-methoxy-4-n-butylaminosulfonyl-phenyl)-6,7-dihydro-3H,5H-pyrrolo-[2,3-f]benzimidazol-6-on

7,7-Dimethyl-2-[2-methoxy-4-(4-morpholinylsulfonyl)-phenyl]-6,7-dihydro-3H,5H-pyrrolo-[2,3-f]benzimidazol-6-on

7,7-Dimethyl-2-(2-methoxy-4-methylsulfenylmethyl-phenyl)-6,7-dihydro-3H,5H-pyrrolo-[2,3-f]benzimidazol-6-on

7,7-Dimethyl-2-(2-methoxy-4-methylsulfinylmethyl-phenyl)-6,7-dihydro-3H,5H-pyrrolo-[2,3-f]benzimidazol-6-on

7,7-Dimethyl-2-(2-methoxy-4-nitro-phenyl)-6,7-dihydro-3H,5H-pyrrolo[2,3-f]benzimidazol-6-on

7,7-Dimethyl-2-(2-methoxy-4-N-methylsulfonylamino-phenyl)-6,7-dihydro-3H,5H-pyrrolo[2,3-f]benzimidazol-6-on

7,7-Dimethyl-2-(2-methoxy-4-methylsulfinyl-phenyl)-6,7-dihydro-3H,5H-pyrrolo[2,3-f]benzimidazol-6-on, Mp. 217–220 °C

7,7-Dimethyl-2-(4-trifluormethyl-phenyl)-6,7-dihydro-3H,5H-pyrrolo[2,3-f]benzimidazol-6-on, Mp. > 300 °C

7,7-Dimethyl-2-(2-methoxy-4-methylsulfonylmethyl-phenyl)-6,7-dihydro-3H,5H-pyrrolo-[2,3-f]benzimidazol-6-on

7,7-Dimethyl-2-(2-methoxy-4-methylaminosulfonyl-phenyl)-6,7-dihydro-3H,5H-pyrrolo-[2,3-f]benzimidazol-6-on

7,7-Dimethyl-2-(2-methoxy-4-propargyloxy-phenyl)-6,7-dihydro-3H,5H-pyrrolo[2,3-f]benz-

imidazol-6-on
7,7-Dimethyl-2-(2-methoxy-4-cyanmethyloxy-phenyl)-6,7-dihydro-3H,5H-pyrrolo[2,3-f]benz-imidazol-6-on
7,7-Dimethyl-2-(2-methoxy-4-methoxycarbonylmethyloxy-phenyl)-6,7-dihydro-3H,5H-pyrrolo[2,3-f]benzimidazol-6-on
7,7-Dimethyl-2-(3,4,5-trimethoxy-phenyl)-6,7-dihydro-3H,5H-pyrrolo[2,3-f]benzimidazol-6-on
7,7-Diethyl-2-phenyl-6,7-dihydro-3H,5H-pyrrolo[2,3-f]benzimidazol-6-on
7,7-Diethyl-2-[4-(1H-imidazol-1-yl)-phenyl]-6,7-dihydro-3H,5H-pyrrolo[2,3-f]benzimidazol-6-on
7,7-Diethyl-2-(2,4-dimethoxy-phenyl)-6,7-dihydro-3H,5H-pyrrolo[2,3-f]benzimidazol-6-on
7,7-Diethyl-2-(2-methoxy-4-hydroxy-phenyl)-6,7-dihydro-3H,5H-pyrrolo[2,3-f]benzimidazol-6-on
7,7-Diethyl-2-(2-methoxy-4-methylsulfonyloxy-phenyl)-6,7-dihydro-3H,5H-pyrrolo[2,3-f]benz-imidazol-6-on
7,7-Diethyl-2-(2-methoxy-4-trifluormethylsulfonyloxy-phenyl)-6,7-dihydro-3H,5H-pyrrolo[2,3-f]benzimidazol-6-on
7,7-Diethyl-2-(2-methoxy-4-methylsulfonyl-amino-phenyl)-6,7-dihydro-3H,5H-pyrrolo-[2,3-f]benzimidazol-6-on
7,7-Diethyl-2-(2-methoxy-4-methylsulfonylmethyl-phenyl)-6,7-dihydro-3H,5H-pyrrolo-[2,3-f]benzimidazol-6-on
7,7-Diethyl-2-(2-methoxy-4-cyan-phenyl)-6,7-dihydro-3H,5H-pyrrolo[2,3-f]benzimidazol-6-on
2′-Phenyl-spiro[cyclopentan-1,7′-6′,7′-dihydro-3′H,5′H-pyrrolo[2′,3′-f]benzimidazol]-6′-on
2′-(2-Methoxy-4-methylsulfonylamino-phenyl)-spiro[cyclopentan-1,7′-6′,7′-dihydro-3′H,5′H-pyrrolo[2′,3′-f]benzimidazol]-6′-on
2′-(2-Methoxy-4-trifluormethylsulfonyloxy-phenyl)spiro[cyclopentan-1,7′-6′,7′-dihydro-3′H,5′H-pyrrolo[2′,3′-f]benzimidazol]-6′-on
2′-(2-Methoxy-4-nitro-phenyl)spiro[cyclopentan-1,7′-6′,7′-dihydro-3′H,5′H-pyrrolo-[2′,3′-f]benzimidazol]-6′-on
2′-(2-Methoxy-4-hydroxy-phenyl)spiro-[cyclopentan-1,7′-6′,7′-dihydro-3′H,5′H-pyrrolo[2′,3′-f]benzimidazol]-6′-on
2′-(2-Methoxy-4-amino-phenyl)spiro[cyclopentan-1,7′-6′,7′-dihydro-3′H,5′H-pyrrolo-[2′,3′-f]benzimidazol]-6′-on
2′-(2-Methoxy-4-methylsulfonyloxy-phenyl)-spiro[cyclopentan-1,7′-6′,7′-dihydro-3′H,5′H-pyrrolo[2′,3′-f]benzimidazol]-6′-on
7-Methyl-2-phenyl-6,7-dihydro-3H,5H-pyrrolo[2,3-f]benzimidazol-6-on
7-Methyl-2-[4-(1H-imidazol-1-yl)phenyl]-6,7-dihydro-3H,5H-pyrrolo[2,3-f]benzimidazol-6-on
7-Methyl-2-(2-methoxy-4-methylsulfonylamino-phenyl)-6,7-dihydro-3H,5H-pyrrolo[2,3-f]benz-imidazol-6-on
7-Methyl-2-(2-methoxy-4-ethylsulfonyloxy-phenyl)-6,7-dihydro-3H,5H-pyrrolo[2,3-f]benz-imidazol-6-on
7-Methyl-2-(2-methoxy-4-trifluormethylsulfonyloxy-phenyl)-6,7-dihydro-3H,5H-pyrrolo[2,3-f]benzimidazol-6-on
7-Methyl-2-(4-dimethylamino-phenyl)-6,7-dihydro-3H,5H-pyrrolo[2,3-f]benzimidazol-6-on
7-Methyl-2-(2-dimethylamino-4-nitro-phenyl)-6,7-dihydro-3H,5H-pyrrolo[2,3-f]benzimidazol-6-on
7-Methyl-2-(2-methoxy-4-nitro-phenyl)-6,7-dihydro-3H,5H-pyrrolo[2,3-f]benzimidazol-6-on
7-Methyl-2-(2-methoxy-4-hydroxy-phenyl)-6,7-dihydro-3H,5H-pyrrolo[2,3-f]benzimidazol-6-on
7-Methyl-2-(2-methoxy-4-aminocarbonylamino-phenyl)-6,7-dihydro-3H,5H-pyrrolo[2,3-f]benz-imidazol-6-on
7-Methyl-2-(2-methoxy-4-cyan-phenyl)-6,7-dihydro-3H,5H-pyrrolo[2,3-f]benzimidazol-6-on
7-Methyl-2-(2-methoxy-4-methylsulfonyloxy-phenyl)-6,7-dihydro-3H,5H-pyrrolo[2,3-f]benz-imidazol-6-on
7-Methyl-2-(2-methoxy-4-methylsulfinylmethyl-phenyl)-6,7-dihydro-3H,5H-pyrrolo[2,3-f]benz-imidazol-6-on
7-Methyl-2-(2-methoxy-4-aminosulfonyl-phenyl)-6,7-dihydro-3H,5H-pyrrolo[2,3-f]benz-imidazol-6-on
7-Methyl-2-(2-methoxy-4-methylaminosulfonyl-phenyl)-6,7-dihydro-3H,5H-pyrrolo-[2,3-f]benzimidazol-6-on
7-Ethyl-2-phenyl-6,7-dihydro-3H,5H-pyrrolo-[2,3-f]benzimidazol-6-on
7-Ethyl-2-[4-(1H-imidazol-1-yl)-phenyl]-6,7-dihydro-3H,5H-pyrrolo[2,3-f]benzimidazol-6-on
7-Ethyl-2-(2-methoxy-4-methylsulfonylamino-phenyl)-6,7-dihydro-3H,5H-pyrrolo[2,3-f]benz-imidazol-6-on
7-Ethyl-2-(2-methoxy-4-N-methyl-methyl-sulfonylamino-phenyl)-6,7-dihydro-3H,5H-pyrrolo[2,3-f]benzimidazol-6-on
7-Ethyl-2-(2-methoxy-4-methylsulfonylmethyl-phenyl)-6,7-dihydro-3H,5H-pyrrolo[2,3-f]benz-imidazol-6-on
7-Ethyl-2-(2-methoxy-4-ethylsulfonyloxy-phenyl)-6,7-dihydro-3H,5H-pyrrolo[2,3-f]-benzimidazol-6-on
7-Ethyl-2-(3-methoxy-4-methylsulfonylamino-phenyl)-6,7-dihydro-3H,5H-pyrrolo[2,3-f]benz-imidazol-6-on
7-Ethyl-2-(2-methoxy-4-trifluormethylsulfonyloxy-phenyl)-6,7-dihydro-3H,5H-pyrrolo[2,3-f]-benzimidazol-6-on
7-Ethyl-2-(2-methoxy-4-nitro-phenyl)-6,7-dihydro-3H,5H-pyrrolo[2,3-f]benzimidazol-6-on
7-Ethyl-2-(3,4-dimethoxy-phenyl-vinyl)-6,7-dihydro-3H,5H-pyrrolo[2,3-f]benzimidazol-6-on
7-Ethyl-2-(2-methoxy-4-hydroxy-phenyl)-6,7-dihydro-3H,5H-pyrrolo[2,3-f]benzimidazol-6-on
7-Ethyl-2-(3,4-dichlor-phenyl)-6,7-dihydro-3H,5H-pyrrolo[2,3-f]benzimidazol-6-on
7-Ethyl-2-(2-methoxy-4-methylaminocarbonyl-amino-phenyl)-6,7-dihydro-3H,5H-pyrrolo[2,3-f]-benzimidazol-6-on
7-Ethyl-2-(2-methoxy-4-methylsulfonyloxy-phenyl)-6,7-dihydro-3H,5H-pyrrolo[2,3-f]-benzimidazol-6-on
7-Isopropyl-2-phenyl-6,7-dihydro-3H,5H-pyrrolo[2,3-f]benzimidazol-6-on
7-Isopropyl-2-[4-(1H-imidazol-1-yl)-phenyl]-6,7-dihydro-3H,5H-pyrrolo[2,3-f]benzimidazol-6-on

7-Isopropyl-2-(2-methoxy-4-methylsulfonyloxy-phenyl)-6,7-dihydro-3H,5H-pyrrolo[2,3-f]benzimidazol-6-on
7-Isopropyl-2-(2-methoxy-4-methylamino-carbonylamino-phenyl)-6,7-dihydro-3H,5H-pyrrolo[2,3-f]benzimidazol-6-on
7-Isopropyl-2-(2-methoxy-4-methylsulfonyl-amino-phenyl)-6,7-dihydro-3H,5H-pyrrolo-[2,3-f]benzimidazol-6-on
7-Isopropyl-2-(2-methoxy-4-nitro-phenyl)-6,7-dihydro-3H,5H-pyrrolo[2,3-f]benzimidazol-6-on
7-Isopropyl-2-(2-methoxy-4-amino-phenyl)-6,7-dihydro-3H,5H-pyrrolo[2,3-f]benzimidazol-6-on
7-Isopropyl-2-(2-methoxy-4-hydroxy-phenyl)-6,7-dihydro-3H,5H-pyrrolo[2,3-f]benzimidazol-6-on
7-Isopropyliden-2-(2-methoxy-4-hydroxy-phenyl)-6,7-dihydro-3H,5H-pyrrolo[2,3-f]-benzimidazol-6-on
7-Acetyl-2-(4-methoxy-phenyl)-6,7-dihydro-3H,5H-pyrrolo[2,3-f]benzimidazol-6-on
7-Acetyl-2-(2-methoxy-4-methylsulfonyloxy-phenyl)-6,7-dihydro-3H,5H-pyrrolo[2,3-f]benzimidazol-6-on
7-Acetyl-2-(2-methoxy-4-methylsulfonylamino-phenyl)-6,7-dihydro-3H,5H-pyrrolo[2,3-f]benzimidazol-6-on
7-Acetyl-7-methyl-2-(4-methoxy-phenyl)-6,7-dihydro-3H,5H-pyrrolo[2,3-f]benzimidazol-6-on
7-Acetyl-7-methyl-2-(2-methoxy-4-hydroxy-phenyl)-6,7-dihydro-3H,5H-pyrrolo[2,3-f]-benzimidazol-6-on
7-Acetyl-7-methyl-2-(2-methoxy-4-methyl-sulfonylamino-phenyl)-6,7-dihydro-3H,5H-pyrrolo[2,3-f]benzimidazol-6-on
7-Acetyl-7-methyl-2-(2-methoxy-4-methyl-sulfonyloxy-phenyl)-6,7-dihydro-3H,5H-pyrrolo[2,3-f]benzimidazol-6-on
7-Methoxycarbonyl-2-(4-methoxy-phenyl)-6,7-dihydro-3H,5H-pyrrolo[2,3-f]benzimidazol-6-on
7-Methoxycarbonyl-2-(2-methoxy-4-methyl-sulfonyloxy-phenyl)-6,7-dihydro-3H,5H-pyrrolo[2,3-f]benzimidazol-6-on
7-Methoxycarbonyl-2-(2-methoxy-4-methyl-sulfonylamino-phenyl)-6,7-dihydro-3H,5H-pyrrolo[2,3-f]benzimidazol-6-on
7-Ethoxycarbonyl-7-ethyl-2-(4-methoxy-phenyl)-6,7-dihydro-3H,5H-pyrrolo[2,3-f]benzimidazol-6-on
7-Ethoxycarbonyl-7-ethyl-2-(2-methoxy-4-methylsulfonyloxy-phenyl)-6,7-dihydro-3H,5H-pyrrolo[2,3-f]benzimidazol-6-on
7-Ethoxycarbonyl-7-ethyl-2-(2-methoxy-4-methylsulfonylamino-phenyl)-6,7-dihydro-3H,5H-pyrrolo[2,3-f]benzimidazol-6-on
7-Ethoxycarbonyl-7-methyl-2-[4-(1H-imidazol-1-yl)-phenyl]-6,7-dihydro-3H,5H-pyrrolo[2,3-f]-benzimidazol-6-on
7-Ethoxycarbonyl-7-methyl-2-(2-methoxy-4-hydroxy-phenyl)-6,7-dihydro-3H,5H-pyrrolo-[2,3-f]benzimidazol-6-on
7-Ethoxycarbonyl-7-methyl-2-(2-methoxy-4-methylsulfonylamino-phenyl)-6,7-dihydro-3H,5H-pyrrolo[2,3-f]benzimidazol-6-on
7-Ethoxycarbonyl-7-methyl-2-(2-methoxy-4-methylsulfonyloxy-phenyl)-6,7-dihydro-3H,5H-pyrrolo[2,3-f]benzimidazol-6-on
7-Aminocarbonyl-7-methyl-2-(4-methoxy-phenyl)-6,7-dihydro-3H,5H-pyrrolo[2,3-f]-benzimidazol-6-on
7-Aminocarbonyl-7-methyl-2-(2-methoxy-4-methylsulfonyloxy-phenyl)-6,7-dihydro-3H,5H-pyrrolo[2,3-f]benzimidazol-6-on
7-Aminocarbonyl-7-methyl-2-(2-methoxy-4-methylsulfonylamino-phenyl)-6,7-dihydro-3H,5H-pyrrolo[2,3-f]benzimidazol-6-on
7-Cyan-7-methyl-2-(4-methoxy-phenyl)-6,7-dihydro-3H,5H-pyrrolo[2,3-f]benzimidazol-6-on
7-Cyan-7-methyl-2-(2-methoxy-4-methyl-sulfonyloxy-phenyl)-6,7-dihydro-3H,5H-pyrrolo[2,3-f]benzimidazol-6-on
7-Cyan-7-methyl-2-(2methoxy-4-methyl-sulfonylamino-phenyl)-6,7-dihydro-3H,5H-pyrrolo[2,3-f]benzimidazol-6-on
7-Hydrazinocarbonyl-7-methyl-2-(4-methoxy-phenyl)-6,7-dihydro-3H,5H-pyrrolo[2,3-f]benzimidazol-6-on

Beispiel 1

7-Isopropyl-2-(4-methoxy-phenyl)-6,7-dihydro-3H,5H-pyrrolo[2,3-f]benzimidazol-6-on

2,8 g (13,6 mmol) 5,6-Diamino-3-isopropyl-indolin-2-on-dihydrochlorid wurden in 10 ml $CH_2Cl_2$ suspendiert, mit 2,55 g (15 mmol) p-Methoxybenzoylchlorid versetzt und bei 25 °C 4,55 g (45 mmol) Triethylamin zugetropft. Die Mischung wurde 2 h gerührt, eingeengt und mit Wasser durchgearbeitet. Der Rückstand wurde in 75 ml Ethanol und 75 ml conc. Salzsäure ca. 24 h zum Rückfluss erhitzt, eingeengt und mit 2 N Ammoniak neutral gestellt. Der abgesaugte Rückstand wurde aus Essigester/Methanol umkristallisiert.

Ausbeute: 2.1 g (48,2% d.Th.), Mp. 326–328 °C.

Analog zu Beispiel 1 erhält man:

| Bezeichnung | Ausbeute (%) | Mp. (°C) Lsg.-mittel |
|---|---|---|
| 7,7-Dimethyl-2-phenylmethyl-6,7-dihydro-3H,5H-pyrrolo(2,3-f)benzimidazol-6-on<br>aus<br>5,6-Diamino-3,3-dimethyl-indolin-2-on<br>und<br>Phenylacetylchlorid | 45 | 328-30, Ethanol/ Wasser |

| Bezeichnung | Ausbeute (%) | Mp. (°C) Lsg.-mittel |
|---|---|---|
| 7,7-Dimethyl-2-(2-methoxy-phenyl)-6,7-dihydro-3H,5H-pyrrolo(2,3-f)benzimidazol-6-on<br>aus<br>5,6-Diamino-3,3-dimethyl-indolin-2-on<br>und<br>2-Methoxybenzoylchlorid | 23 | 300-03, Aceton |
| 7,7-Dimethyl-2-(4-methoxy-phenyl)-6,7-dihydro-3H,5H-pyrrolo(2,3-f)benzimidazol-6-on<br>aus<br>5,6-Diamino-3,3-dimethyl-indolin-2-on<br>und<br>4-Methoxybenzoylchlorid | 46 | 341-43, Dioxan/ Wasser, 2:1 |
| 7,7-Dimethyl-2-(4-chlor-phenyl)-6,7-dihydro-3H,5H-pyrrolo(2,3-f)benzimidazol-6-on<br>aus<br>5,6-Diamino-3,3-dimethyl-indolin-2-on<br>und<br>4-Chlorbenzoylchlorid | 11 | 360–63, Aceton |
| 7,7-Dimethyl-2-(2-methoxy-5-methyl-sulfonyl-phenyl)-6,7-dihydro-3H,5H-pyrrolo(2,3-f)benzimidazol-6-on<br>aus<br>5,6-Diamino-3,3-dimethyl-indolin-2-on<br>und<br>2-Methoxy-5-methylsulfonyl-benzoylchlorid | 29 | 236–38, Ethanol/ Wasser |
| 7,7-Dimethyl-2-(4-methyl-phenyl)-6,7-dihydro-3H,5H-pyrrolo(2,3-f)benzimidazol-6-on<br>aus<br>5,6-Diamino-3,3-dimethyl-indolin-2-on<br>und<br>4-Methylbenzoylchlorid | 30 | > 300, Isopropanol |
| 7,7-Diethyl-2-(4-methoxy-phenyl)-6,7-dihydro-3H,5H-pyrrolo(2,3-f)-benzimidazol-6-on<br>aus<br>5,6-Diamino-3,3-diethyl-indolin-2-on<br>und<br>4-Methoxybenzoylchlorid | 44 | 217–19 Essigsäure-ethylester |
| 2′-(4-Methoxy-phenyl)-spiro(cyclopentan-1,7′-6′-7′-dihydro-3′H,5′H-pyrrolo(2′,3′-f)benzimidazol)-6′-on<br>aus<br>5′,6′-Diamino-spiro(cyclopentan-1,3′-indolin)-2′-on und<br>4-Methoxybenzoylchlorid | 37 | 354–55 Dioxan/Wasser, 1:1 |
| 7-Methyl-2-(4-methoxy-phenyl)-6,7-dihydro-3H,5H-pyrrolo(2,3-f)benzimidazol-6-on<br>aus<br>5,6-Diamino-3-methyl-indolin-2-on-dihydrochlorid<br>und<br>4-Methoxybenzoylchlorid | 43 | > 300 Essigester/ Methanol |
| 7-Methyl-2-(2,4-Dimethoxy-phenyl)-6,7-dihydro-3H,5H-pyrrolo(2,3-f)-benzimidazol-6-on<br>aus<br>5,6-Diamino-3-methyl-indolin-2-on dihydrochlorid<br>und<br>2,4-Dimethoxybenzoylchlorid | 12 | 294–97 Essigester/ Methanol |

Beispiel 2

7,7-Dimethyl-2-(4-hydroxy-phenyl)-6,7-dihydro-3H,5H-pyrrolo[2,3-f]benzimidazol-6-on

Eine Lösung von 3,6 g (0,019 mol) 5,6-Diamino-3,3-dimethyloxindol und 4,0 g (0,019 mol) 4-Benzyloxybenzaldehyd werden in 100 ml Ethanol unter Durchleiten von Luft zum Sieden erhitzt. Nach 1 h wird abgekühlt und der ausgefallene Niederschlag abgesaugt. Man erhält 5,4 g (74%) farblose Kristalle vom Mp. 250°C.

5,4 g (0,014 mol) dieser Verbindung werden in 200 ml Methanol in Gegenwart von 0,5 g 10% Pd auf Kohle bei Normaldruck hydriert. Der Katalysator wird abgesaugt und das Lösungsmittel i. Vak. eingeengt.

Man erhält 1,8 g (44%) der Titelverbindung als farblose Kristalle mit Mp. 240°C (Aceton).

Beispiel 3

7,7-Dimethyl-2-phenyl-6,7-dihydro-3H,5H-pyrrolo[2,3-f]benzimidazol-6-on

a) In eine Lösung von 5,5 g (25 mmol) 6-Amino-5-nitro-3,3-dimethyl-indolin-2-on in 50 ml Pyridin werden unter Eiskühlung 5,6 ml (50 mmol) Benzoylchlorid zugetropft und bei 25°C nachgerührt. Der Kristallbrei wird auf ca. 300 ml Wasser gegossen, abgesaugt und mit Wasser gewaschen und getrocknet.

Ausbeute: 10,7 g 6-Benzoylamino-5-nitro-3,3-dimethyl-indolin-2-on

b) Zu 10,0 g (30,7 mmol) 6-Benzoylamino-5-nitro-3,3-dimethyl-indolin-2-on in 250 ml Eisessig werden 1,0 g Palladium auf Kohle (10proz.) zugesetzt. Diese Mischung wird in einer Schüttelente bei Zimmertemperatur und Normaldruck hydriert. Nach beendeter Wasserstoffaufnahme wird die Lösung vom Katalysator abgetrennt, langsam bei 60°C eingeengt und der in Wasser aufgenommene Rückstand mit 2 N wässrigem Ammoniak neutralisiert. Der Rückstand wird abgesaugt und aus Methylenchlorid umkristallisiert.

Ausbeute: 6,9 g (81% d. Th.) Mp. 195–220°C

Analog zu Beispiel 3 erhält man:

| Bezeichnung | Ausbeute (%) | Mp. (°C) Lsg.-mittel |
|---|---|---|
| 7,7-Dimethyl-2-(2,4-dimethoxy-phenyl)-6,7-dihydro-3H,5H-pyrrolo-(2,3-f)benzimidazol-6-on aus 5-(2,4-Dimethoxy-benzoylamino)-6-nitro-3,3-dimethyl-indolin-2-on | 25 | 294–97, Dioxan |
| 7,7-Dimethyl-2-(3,4-dimethoxy-phenyl)-6,7-dihydro-3H,5H-pyrrolo(2,3-f)benzimidazol-6-on aus 6-(3,4-Dimethoxy-benzoylamino)-5-nitro-3,3-dimethyl-indolin-2-on | 75 | 314–17, Ethanol |
| 7,7-Dimethyl-2-(2-methoxy-4-chlor-phenyl)-6,7-dihydro-3H,5H-pyrrolo(2,3-f)benzimidazol-6-on aus 6-(2-methoxy-4-chlor-benzoylamino)-5-nitro-3,3-dimethyl-indolin-2-on | 12 | 299–301, Ethanol |

Beispiel 4

7-Methyl-2-(2-methoxy-4-amino-phenyl)-6,7-dihydro-3H,5H-pyrrolo[2,3-f]benzimidazol-6-on-hydrochlorid

a) 3,5 g (14 mmol) 5,6-Diamino-3-methyl-indolin-2-on-dihydrochlorid, 4,5 g (21 mmol) 2-methoxy-4-nitro-benzoylchlorid und 6,95 ml (49 mmol) Triethylamin werden in 50 ml $CH_2Cl_2$ 3 h bei Raumtemperatur gerührt. Anschliessend wird zur Trockne destilliert, mit Wasser durchgearbeitet und aus Methanol/Methylenchlorid umkristallisiert. Man erhält 3 g eines Gemisches aus Mono- und Diamid.

b) 2,6 g des so erhaltenen Produktes werden in 400 ml Methanol mit 0,8 g 10proz. Palladium/Kohle hydriert. Nach beendeter Wasserstoffaufnahme wurde vom Katalysator abgesaugt, das Methanol abdestilliert und der Rückstand mit 70 ml Ethanol und 35 ml konzentrierter Salzsäure 24 Stunden gekocht. Das Lösungsmittel wurde abdestilliert und der ölige Rückstand aus Ethanol kristallisiert. Man erhält 1,1 g der Titelverbindung vom Mp. 280–310°C.

Analog zu Beispiel 4 erhält man:

| Bezeichnung | Ausbeute (%) | Mp. (°C) Lsg.-mittel |
|---|---|---|
| 7,7-Dimethyl-2-(2-methoxy-4-amino-phenyl)-6,7-dihydro-3H,5H-pyrrolo(2,3-f)benzimidazol-6-on-hydrochlorid | 18 | 295, Ethanol |
| 7-Ethyl-2-(2-methoxy-4-amino-phenyl)-6,7-dihydro-3H,5H-pyrrolo(2,3-f)benzimidazol-6-on-hydrochlorid | 10 | 274, Ethanol |

Beispiel 5

7-Ethyl-2-(2-methoxy-4-acetylamino-phenyl)-6,7-dihydro-3H,5H-pyrrolo[2,3-f]benzimidazol-6-on

0,7 g (1,8 mmol) 7-Ethyl-2-(2-methoxy-4-amino-phenyl)-6,7-dihydro-3H,5H-pyrrolo[2,3-f]benzimidazol-6-on-hydrochlorid, 15 ml Acetanhydrid und 0,3 g Natriumacetat wurden 3 h bei 40°C gerührt. Nach dem Abkühlen wurde abgesaugt und nach Behandeln mit Kohle aus Methanol kristallisiert.

Ausbeute: 0,44 g (69%), Mp. 245–49°C.

Beispiel 6

7,7-Dimethyl-2-[4-(1H-imidazol-1-yl)-phenyl]-6,7-dihydro-3H,5H-pyrrolo[2,3-f]benzimidazol-6-on

2,0 g (10,4 mmol) 5,6-Diamino-3,3-dimethyl-indolin-2-on, 1,8 g (10,4 mmol) 4-(1H-imidazol-1-yl)-benzaldehyd, 0,2 g (1 mmol) p-Toluolsulfonsäure und 120 ml Ethanol werden zusammengegeben und nach Erreichen des Siedepunktes 3 Stunden Luft durchgeleitet. Nach Einengen und Ansäuern mit ethanolischer Salzsäure wird das Kristallisat abgesaugt, in Wasser suspendiert und mit wässrigem Ammoniak neutralisiert. Der Rückstand wurde aus Methanol umkristallisiert.

Ausbeute: 1,0 g (28%), Mp. 300°C.

Analog zu Beispiel 6 erhält man:

| Bezeichnung | Ausbeute (%) | Mp. (°C) Lsg.-mittel |
|---|---|---|
| 7,7-Dimethyl-2-(4-dimethylamino-phenyl)-6,7-dihydro-3H,5H-pyrrolo(2,3-f)benzimidazol-6-on-hydrochlorid<br>aus<br>5,6-Diamino-3,3-dimethyl-indolin-2-on<br>und<br>4-Dimethylaminobenzaldehyd | 23 | 262–70, Isopropanol |

Beispiel 7

7,7-Dimethyl-2-(2-methoxy-4-methylmercapto-phenyl)-6,7-dihydro-3H,5H-pyrrolo[2,3-f]benzimidazol-6-on

In einer Lösung von 5,5 g (25 mmol) 6-Amino-5-nitro-3,3-dimethyl-indolin-2-on und 9,9 g (50 mmol) 2-Methoxy-4-methylmercapto-benzoesäure in 50 ml Pyridin werden 7,65 g (50 mmol) $POCl_3$ bei 25°C zugetropft. Die Aufarbeitung und nachfolgende Hydrierung erfolgt analog Beispiel 3.

Ausbeute: 5,3 g (60%) Mp. 293–295°C.

Beispiel 8

7,7-Dimethyl-2-(2-methoxy-4-methylsulfonylamino-phenyl)-6,7-dihydro-3H,5H-pyrrolo-[2,3-f]benzimidazol-6-on

Zu einer Lösung von 3,6 g (10 mmol) 7,7-Dimethyl-2-(2-methoxy-4-amino-phenyl)-6,7-dihydro-3H,5H-pyrrolo[2,3-f]benzimidazol-6-on-hydrochlorid und 2,2 g (22 mmol) Triethylamin in 50 ml Dimethylformamid werden langsam 1,26 g (11 mmol) Methansulfonylchlorid zugetropft. Nach 2 h Rühren bei 25°C wird das Dimethylformamid im Hochvakuum abdestilliert, der Rückstand mit Wasser durchgearbeitet und abgesaugt.

Anschliessend wurde aus Ethanol umkristallisiert.

Ausbeute: 2,9 g (72,5%), Mp. >300°C.

Beispiel 9

7,7-Dimethyl-2-(2-methoxy-4-cyan-phenyl)-6,7-dihydro-3H,5H-pyrrolo[2,3-f]benzimidazol-6-on

Zu 17,7 g 4-cyano-2-methoxy-benzoesäure (Schmp. 170–173°C, 0,1 mol) in 180 ml Dichlormethan gab man unter Kühlung portionsweise 22,9 g (0,11 mol) $PCl_5$. Das Lösungsmittel wurde i. Vak. entfernt und der Rückstand (Säurechlorid) ohne weitere Reinigung eingesetzt.

9,8 g (0,05 mol) dieses Rückstandes werden in 50 ml Dichlormethan aufgenommen, 6,9 ml (0,05 mol) Triethylamin und 5,7 g (0,03 mol) 5,6-Diamino-3,3-dimethyloxindol zugegeben und bei Raumtemperatur gerührt. Nach Filtration wird das Lösungsmittel i. Vak. entfernt und der Rückstand ohne weitere Reinigung eingesetzt.

5,3 g des Rückstandes werden in 200 ml Ethanol und 30 ml konzentrierter Salzsäure 3 Stunden zum Rückfluss erhitzt. Das Lösungsmittel wird im Vakuum entfernt, und der Rückstand mit wässriger Ammoniaklösung digeriert. Nach chromatographischer Reinigung (Kieselgel, Dichlormethan:methanol. Ammoniak: 20:1 und Toluol:Essigester:Essigsäure = 5:5:1) erhält man 1,4 g der Titelverbindung als Acetat mit Mp. 304–312°C.

Beispiel 10

7,7-Dimethyl-2-(2-methoxy-4-carboxy-phenyl)-6,7-dihydro-3H,5H-pyrrolo[2,3-f]-benzimidazol-6-on

0,4 g (1,2 mmol) 3,3-Dimethyl-2-(2-methoxy-4-cyan-phenyl)-6,7-dihydro-3H,5H-pyrrolo[2,3-f]-benzimidazol-6-on werden mit 4 ml 2 N Natronlauge eine Stunde zum Rückfluss erhitzt. Anschliessend wird abgekühlt, mit Eisessig angesäuert und das Kristallisat abgesaugt.

Ausbeute: 0,4 g (95%), Mp. 315–19°C.

Beispiel 11

7,7-Dimethyl-2-(2-methoxy-4-aminocarbonyl-phenyl)-6,7-dihydro-3H,5H-pyrrolo[2,3-f]benzimidazol-6-on

0,25 g (0,71 mmol) 7,7-Dimethyl-2-(2-methoxy-4-carboxy-phenyl)-6,7-dihydro-3H,5H-pyrrolo-

[2,3-f]benzimidazol-6-on werden mit 2 ml Thionylchlorid drei Stunden am Rückfluss gekocht und anschliessend zur Trockne eingedampft. Das Säurechlorid wird in 2 ml Dioxan suspendiert und 2 ml konzentrierter Ammoniak zugetropft. Man rührt 1 Stunde bei 80°C, destilliert das Dioxan und Wasser ab, verrührt den Rückstand mit Wasser und saugt ab.

Ausbeute: 0,15 g (60%), Mp. 265–270°C.

Beispiel 12

7,7-Dimethyl-2-(2-methoxy-4-ethoxycarbonyl-phenyl)-6,7-dihydro-3H,5H-pyrrolo[2,3-f]benzimidazol-6-on

0,25 g (0,71 mmol) 7,7-Dimethyl-2-(2-methoxy-4-carboxy-phenyl)-6,7-dihydro-3H,5H-pyrrolo-[2,3-f]benzimidazol-6-on werden mit 2 ml Thionylchlorid drei Stunden am Rückfluss gekocht und anschliessend zur Trockne eingedampft. Das Säurechlorid wird mit 5 ml Ethanol versetzt und eine Stunde am Rückfluss gekocht, abgekühlt und das Kristallisat abgesaugt.

Ausbeute: 0,16 g (59%), Mp. 235–240°C.

Beispiel 13

7,7-Dimethyl-2-(2-methoxy-4-hydroxy-phenyl)-6,7-dihydro-3H,5H-pyrrolo[2,3-f]benzimidazol-6-on

Analog Beispiel 1 erhält man aus 2,0 g (10,4 mmol) 5,6-Diamino-3,3-dimethyl-indolin-2-on und 5,75 g (20,8 mmol) 4-Benzyloxy-2-methoxy-benzoylchlorid die Titelverbindung.

Ausbeute: 2,1 g (62,5%), Mp. 225–240°C Zers.

Beispiel 14

7,7-Dimethyl-2-(2-methoxy-4-methylsulfonyl-oxy-phenyl)-6,7-dihydro-3H,5H-pyrrolo[2,3-f]-benzimidazol-6-on

Zu einer Lösung von 1,5 g (4,6 mmol) 7,7-Dimethyl-2-(2-methoxy-4-hydroxy-phenyl)-6,7-dihydro-3H,5H-pyrrolo[2,3-f]benzimidazol-6-on und 0,46 g (4,6 mmol) Triethylamin in 20 ml DMF werden bei 25°C 0,53 g (4,6 mmol) Methansulfonylchlorid zugetropft. Nach 2 h wird das DMF im Vakuum abdestilliert. Der Rückstand wird mit Wasser durchgearbeitet und abgesaugt. Der Rückstand wurde aus Ethanol umkristallisiert.

Ausbeute: 1,3 g (70,6%), Mp. 233–235°C.

Beispiel 15

7-Ethoxycarbonyl-7-methyl-2-phenyl-6,7-dihydro-3H,5H-pyrrolo[2,3-f]benzimidazol-6-on

a) 4 g (14,8 mmol) 3-Ethoxycarbonyl-3-methyl-6-amino-indolin-2-on-hydrochlorid und 2,99 g (29,6 mmol) Triethylamin werden in 60 ml Methylenchlorid suspendiert und unter Eiskühlung 2,08 g (14,8 mmol) Benzoylchlorid zugestropft. Nach zwei Stunden wird das Methylenchlorid abdestilliert, der Rückstand mit Wasser durchgearbeitet, abgesaugt und aus Ethanol kristallisiert. Man erhält 3,5 g (70%) 3-Ethoxycarbonyl-3-methyl-6-benzoylamino-indolin-2-on, Mp. 206–07°C.

b) 3 g (8,86 mmol) der nach a) erhaltenen Verbindung wird in 20 ml konzentrierter Schwefelsäure gelöst. Bei 0–5°C werden 0,985 g (9,75 mmol) $KNO_3$, gelöst in konzentrierter Schwefelsäure, langsam zugetropft. Nach drei Stunden wurde auf Eis gegossen, abgesaugt, der Rückstand in Wasser suspendiert, mit wässrigem Ammoniak neutralisiert, abgesaugt und aus Ethanol umkristallisiert. Man erhält 2,75 g (81%) 3-Ethoxy-carbonyl-3-methyl-5-nitro-6-benzoylamino-indolin-2-on, Mp. 220–23°C.

c) Zu 2,55 g (6,66 mmol) der nach b) erhaltenen Verbindung in 100 ml Eisessig werden 0,5 g Palladium auf Kohle (10proz.) zugesetzt. Diese Suspension wird bei Zimmertemperatur und Normaldruck hydriert. Nach beendeter Wasserstoffaufnahme wird die Lösung vom Katalysator abgetrennt, langsam bei 60°C eingeengt und der in Wasser aufgenommene Rückstand mit 2 N wässrigem Ammoniak neutralisiert und abgesaugt. Nach Umkristallisation aus Ethanol erhält man die Titelverbindung.

Ausbeute: 1,3 g (62%), Mp. 178–82°C.

Beispiel 16

7,7-Dimethyl-2-(2-methoxy-4-methylsulfonyl-phenyl)-6,7-dihydro-3H,5H-pyrrolo[2,3-f]benzimidazol-6-on

200 mg (0,56 mmol) 7,7-Dimethyl-2-(2-methoxy-4-methylmercapto-phenyl)-6,7-dihydro-3H,5H-pyrrolo[2,3-f]benzimidazol-6-on werden 48 h bei Raumtemperatur in 4 ml Eisessig und 0,4 ml 30% $H_2O_2$ gerührt. Man gibt 10 ml Wasser zu und entfernt das Lösungsmittel im Vakuum. Man erhält 160 mg (75%) der Titelverbindung nach Kristallisation aus Ethanol mit MP. 235–237°C.

## Patentansprüche

1. Verbindungen der allgemeinen Formel I

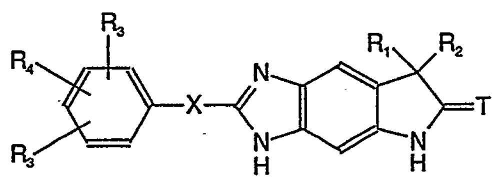 (I),

in welcher

$R_1$ ein Wasserstoffatom, eine $C_1$–$C_6$-Alkyl-, $C_2$–$C_6$-Alkenyl- oder eine $C_3$–$C_7$-Cycloalkylgruppe bedeutet,

$R_2$ ein Wasserstoffatom, eine $C_1$–$C_6$-Alkyl-, $C_2$–$C_6$-Alkenyl- oder Cyangruppe, eine durch eine Hydroxy-, $C_1$–$C_6$-Alkyl-, $C_1$–$C_6$-Alkoxy-, Amino-, $C_1$–$C_6$-Alkylamino-, Di-$C_1$–$C_6$-Alkylamino- oder Hydrazinogruppe substituierte Carbonylgruppe, oder mit $R_1$ zusammen eine $C_3$–$C_7$-Cycloalkylengruppe darstellt oder $R_1$ und $R_2$ zusammen eine $C_3$–$C_7$-Alkyliden- oder $C_3$–$C_7$-Cycloalkylidengruppe bilden,

$R_3$, $R_4$ und $R_5$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine

durch eine Hydroxy-, Alkoxy-, Amino-, Alkylamino- oder Dialkylaminogruppe substituierte Carbonylgruppe, eine durch eine Amino-, Alkylamino- oder Dialkylamino- substituierte Sulfonylgruppe, eine Morpholino-, Pyrrolidino-, Piperidino- oder Hexamethyleniminosulfonylgruppe, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Trifluormethyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl- oder Cyanogruppe sein können, wobei die Alkylteile der oben genannten Substituenten 1-5 Kohlenstoffatome enthalten, und

X ein Valenzstrich, eine $C_1$–$C_4$-Alkylengruppe oder die Vinylengruppe sein kann,

T ein Sauerstoffatom darstellt,

deren Tautomere und deren physiologisch verträgliche Salze anorganischer und organischer Säuren.

2. Verbindungen der allgemeinen Formel I gemäss Anspruch 1

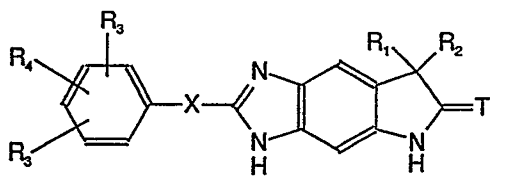

(I),

in der

$R_1$ ein Wasserstoffatom, die Methyl- oder Ethylgruppe darstellt

und $R_2$ die Methyl-, Ethyl-, Isopropyl-, Cyan-, Acetyl-, Methoxycarbonyl-, Ethoxycarbonyl-, Aminocarbonyl- und Hydrazinocarbonylgruppe bedeuten,

$R_1$ und $R_2$ einen Spirocyclopentylring darstellen, wenn $R_1$ und $R_2$ mit dem C-Atom, an das sie gebunden sind, einen $C_3$–$C_7$-Cycloalkylring bilden,

$R_3$ ein Wasserstoff, die Methansulfonyloxy-, Trifluormethansulfonyloxy-, Methansulfonylamino-, Trifluormethansulfonylamino-, Methansulfonylmethylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino- oder die 1-Imidazolylgruppe darstellt,

$R_4$ Wasserstoff, die Methyl-, Methoxy-, Dimethylamino- oder Chlorgruppe bedeutet,

$R_5$ Wasserstoff oder die Methoxygruppe ist,

X ein Valenzstrich, eine Alkylengruppe mit 1 oder 2 Kohlenstoffatomen oder die Vinylengruppe ist und

T für ein Sauerstoffatom steht,

deren Tautomere und deren physiologisch verträgliche Salze anorganischer und organischer Säuren.

3. Verbindungen gemäss Anspruch 1 oder 2, in denen $R_1$ und $R_2$ jeweils eine $C_1$–$C_6$ Alkylgruppe bedeuten, oder $R_1$ und $R_2$ einen Spirocyclopentylring darstellen, $R_3$, $R_4$ und $R_5$ gleich oder verschieden sein können und jeweils Wasserstoff, eine $C_1$–$C_5$ Alkoxy-, Hydroxy- oder 1-Imidazolylgruppe sein können, X ein Valenzstrich und T ein Sauerstoffatom darstellen.

4. Verbindungen gemäss Anspruch 3, in denen $R_1$ und $R_2$ jeweils eine Methylgruppe bedeuten, $R_3$ Wasserstoff, eine Methoxy-, Hydroxy- oder 1-Imidazolylgruppe und $R_4$ und $R_5$ ein Wasserstoffatom darstellen.

5. Die folgenden Verbindungen, die aus der Gruppe der Verbindungen gemäss Anspruch 1 ausgewählt werden:

7,7-Dimethyl-2-(2-methoxy-phenyl)-6,7-dihydro-3H,5H-pyrrolo[2,3-f]benzimidazol-6-on

7,7-Dimethyl-2-(4-hydroxy-phenyl)-6,7-dihydro-3H,5H-pyrrolo[2,3-f]benzimidazol-6-on

7,7-Dimethyl-2-phenyl-6,7-dihydro-3H,5H-pyrrolo[2,3-f]benzimidazol-6-on

7,7-Dimethyl-2-[4-(1H-imidazol-1-yl)-phenyl]-6,7-dihydro-3H,5H-pyrrolo[2,3-f]benzimidazol-6-on

2′-(4-Methoxy-phenyl)-spiro[cyclopentan-1,7′-6′,7′-dihydro-3′H,5′H-pyrrolo[2′,3′-f]benzimidazol-6′-on

7,7-Dimethyl-2-(3,4-dichlorphenyl)-6,7-dihydro-3H,5H-pyrrolo[2,3-f]benzimidazol-6-on

7,7-Dimethyl-2-(4-trifluormethyl-phenyl)-6,7-dihydro-3H,5H-pyrrolo[2,3-f]benzimidazol-6-on

7,7-Dimethyl-2-(2-methoxy-4-chlor-phenyl)-6,7-dihydro-3H,5H-pyrrolo[2,3-f]benzimidazol-6-on

2′-Phenylspiro[cyclopentan-1,7′-6′,7′-dihydro-3′H,5′H-pyrrolo[2′,3′-f]benzimidazol]-6-on.

6. Verfahren zur Herstellung von Verbindungen der allgemeinen Formel I

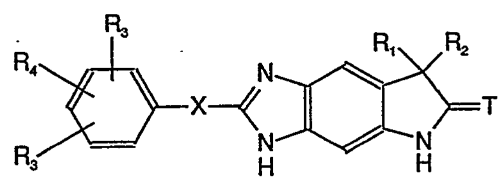

(I),

in welcher

$R_1$ ein Wasserstoffatom, eine $C_1$–$C_6$-Alkyl-, $C_2$–$C_6$-Alkenyl- oder eine $C_3$–$C_7$-Cycloalkylgruppe bedeutet,

$R_2$ ein Wasserstoffatom, eine $C_1$–$C_6$-Alkyl-, $C_2$–$C_6$-Alkenyl- oder Cyangruppe, eine durch eine Hydroxy-, $C_1$–$C_6$-Alkyl-, $C_1$–$C_6$-Alkoxy-, Amino-, $C_1$–$C_6$-Alkylamino-, Di-$C_1$–$C_6$-Alkylamino- oder Hydrazinogruppe substituierte Carbonylgruppe, oder mit $R_1$ zusammen eine $C_3$–$C_7$-Cycloalkylengruppe darstellt oder $R_1$ und $R_2$ zusammen eine $C_3$–$C_7$-Alkyliden- oder $C_3$–$C_7$-Cycloalkylidengruppe bilden,

$R_3$, $R_4$ und $R_5$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino- oder Dialkylamino- substituierte Sulfonylgruppe, eine Morpholino-, Pyrrolidino-, Piperidino- oder Hexamethyleniminosulfonylgruppe, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Trifluormethyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl- oder Cyanogruppe sein können, wobei die Alkylteile der oben genannten Substituenten 1-5 Kohlenstoffatome enthalten, und

X ein Valenzstrich, eine $C_1$-$C_4$-Alkylengruppe oder die Vinylengruppe sein kann,

T ein Sauerstoffatom darstellt,

deren Tautomere und deren physiologisch verträgliche Salze anorganischer und organischer Säuren, dadurch gekennzeichnet, dass man entweder

a) Verbindungen der allgemeinen Formel II

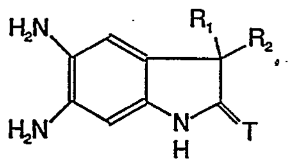

(II),

in der

$R_1$, $R_2$ und T die oben angegebene Bedeutung haben, mit einer Verbindung der allgemeinen Formel III

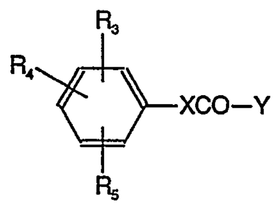

(III),

in der

$R_3$, $R_4$, $R_5$ und X die angegebene Bedeutung besitzen und Y entweder Wasserstoff oder ein leicht abspaltbarer Rest darstellt, umsetzt und die erhaltenen Verbindungen zu einer Verbindung der Formel I oder dessen tautomeren Form cyclisiert oder

b) Verbindungen der allgemeinen Formel IV

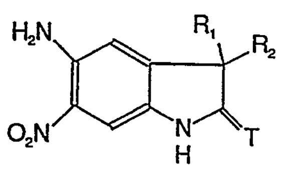

(IV),

oder V,

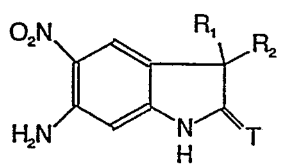

(V),

in denen

$R_1$, $R_2$ und T die angegebene Bedeutung haben, mit einer Verbindung der allgemeinen Formel III, in der

$R_3$, $R_4$, $R_5$ und X die angegebene Bedeutung besitzen und Y ein leicht abspaltbarer Rest darstellt, nach literaturbekannten Verfahren zu Verbindungen der allgemeinen Formel VI,

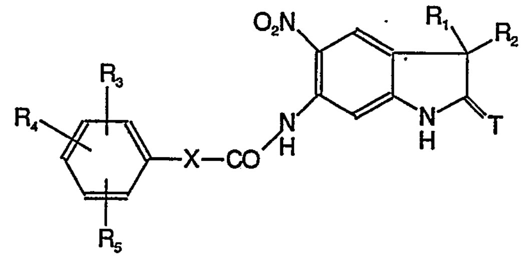

(VI),

oder VII

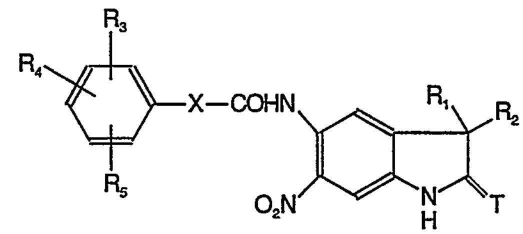

(VII),

in denen

$R_1$, $R_2$, $R_3$, $R_4$, $R_5$, X und T die oben angegebene Bedeutung haben, acyliert, die Nitrogruppe hydriert und die erhaltenen Verbindungen cyclisiert oder

c) ausgehend von Verbindungen der allgemeinen Formel VIII

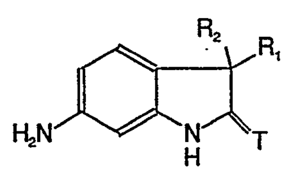

(VIII),

oder IX

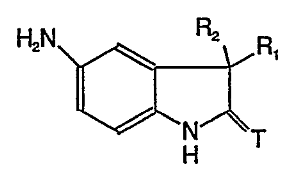

(IX),

in denen $R_1$, $R_2$ und T die oben angegebene Bedeutung besitzen, durch Umsetzung mit Verbindungen der allgemeinen Formel III, in denen Y einen leicht abspaltbaren Rest darstellt, nach allgemein bekannter Weise Verbindungen der Formel X

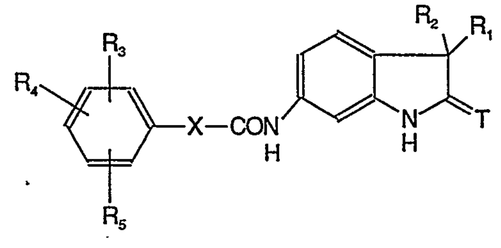

(X),

oder XI

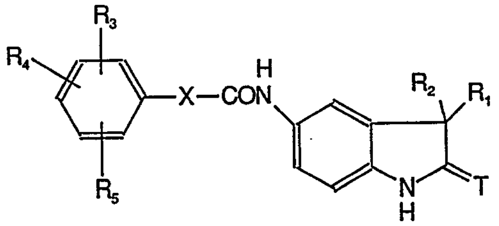

(XI),

in denen $R_1$, $R_2$, $R_3$, $R_4$, $R_5$, T und X die oben angegebenen Bedeutungen besitzen, erhält und in bekannter Weise durch Nitrierung in Verbindungen der allgemeinen Formel VI

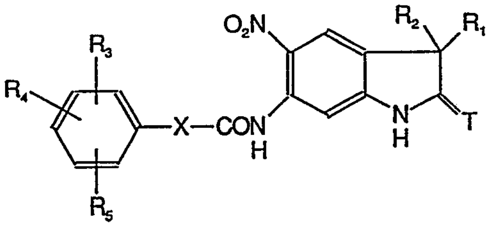

(VI),

oder VII

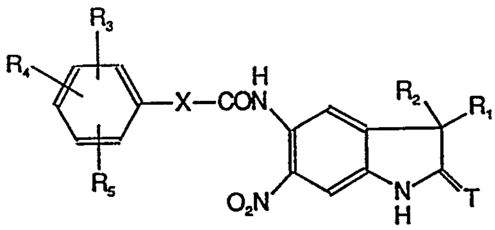

(VII),

in denen $R_1$, $R_2$, $R_3$, $R_4$, $R_5$, T und X die oben angegebenen Bedeutungen haben, überführt, die Nitrogruppe hydriert und die erhaltenen Verbindungen cyclisiert und anschliessend eine erfindungsgemäss erhaltene Verbindung der Formel I gewünschtenfalls in eine andere Verbindung der Formel I oder dessen Tautomer umwandelt und/oder eine erhaltene Verbindung der allgemeinen Formel I und dessen Tautomer in ein physiologisch verträgliches Säureadditionssalz mit einer anorganischen oder organischen Säure überführt.

7. Verfahren gemäss Anspruch 6 zur Herstellung von Verbindungen der allgemeinen Formel I

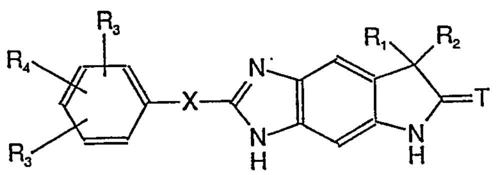

(I),

in der
$R_1$ ein Wasserstoffatom, die Methyl- oder Ethylgruppe darstellt
und $R_2$ die Methyl-, Ethyl-, Isopropyl-, Cyan-, Acetyl-, Methoxycarbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen $C_3$-$C_7$-Cycloalkylring bilden,
$R_3$ ein Wasserstoff, die Methansulfonyloxy-, Trifluormethansulfonyloxy-, Methansulfonylamino-, Trifluormethansulfonylamino-, Methansulfonylmethylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino- oder die 1-Imidazolylgruppe darstellt,
$R_4$ Wasserstoff, die Methyl-, Methoxy-, Dimethylamino- oder Chlorgruppe bedeutet,
$R_5$ Wasserstoff oder die Methoxygruppe ist,
X ein Valenzstrich, eine Alkylengruppe mit 1 oder 2 Kohlenstoffatomen oder die Vinylengruppe ist und
T für ein Sauerstoffatom steht.

8. Arzneimittel, enthaltend eine Verbindung gemäss Anspruch 1 bis 5 neben üblichen Träger- und Hilfsstoffen.

9. Verbindungen gemäss Anspruch 1 bis 5 zur Prophylaxe oder Bekämpfung von Herz- und Kreislauferkrankungen.

10. Verwendung von Verbindungen gemäss Anspruch 1 bis 5 zur Herstellung von Arzneimitteln zur Prophylaxe oder Bekämpfung von Herz- und Kreislauferkrankungen.

## Claims

1. Compounds of the general formula I

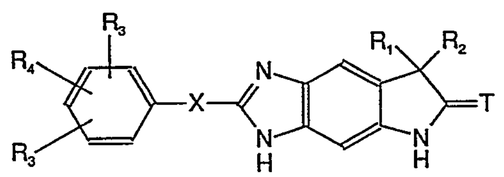

(I),

in which $R_1$ signifies a hydrogen atom, a $C_1$-$C_6$-alkyl, $C_2$-$C_6$-alkenyl or a $C_3$-$C_7$-cycloalkyl group; $R_2$ a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or, together with $R_1$, represents a $C_3$-$C_7$-cycloalkylene radical or $R_1$ and $R_2$ together form a $C_3$-$C_7$-alkylidene or $C_3$-$C_7$-cycloalkylidene group, $R_3$, $R_4$ and $R_5$ can be the same or different and each can be hydrogen, an alkanesulphonyloxy, trifluoromethanesulphonyloxy, alkanesulphonylamino, trifluoromethanesulphonylamino, N-alkylalkanesulphonylamino, N-alkyl-trifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino

group, a sulphonyl group substituted by an amino, alkylamino or dialkylamino, a morpholino, pyrrolidino, piperidino or hexamethyleneiminosulphonyl group, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, trifluoromethyl, alkoxy, alkenyloxy, alkynyloxy, cyanoalkoxy, carboxyalkoxy, alkoxycarbonylalkoxy, dialkylamino, 1-imidazolyl or cyano group, whereby the alkyl moieties of the above-mentioned substituents can contain 1-5 carbon atoms, and X can be a valency bond, a $C_1$–$C_4$-alkylene group or the vinylene group, T represents an oxygen atom; their tautomers thereof and their physiologically acceptable salts of inorganic and organic acids.

2. Compounds of the general formula according to claim 1

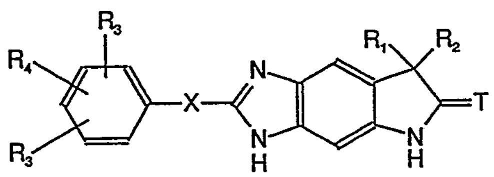
(I),

in which $R_1$ represents a hydrogen atom, the methyl or ethyl group and $R_2$ signifies the methyl, ethyl, isopropyl, cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$, with the C-atom to which they are attached, form a $C_3$–$C_7$-cycloalkyl ring; $R_3$ represents a hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino or the 1-imidazolyl group, $R_4$ signifies hydrogen, the methyl, methoxy, dimethylamino or chlorine group; $R_5$ hydrogen or the methoxy group, X is a valency bond, an alkylene group with 1 or 2 carbon atoms or the vinylene group; and T stands for an oxygen atom; their tautomers and their physiologically acceptable salts of inorganic and organic acids.

3. Compounds according to claim 1 or 2, in which $R_1$ and $R_2$ each signify a $C_1$–$C_6$-alkyl group or $R_1$ and $R_2$ represent a spirocyclopentyl ring, $R_3$, $R_4$ and $R_5$ can be the same or different and each represents hydrogen, a $C_1$–$C_5$-alkoxy, hydroxyl or 1-imidazolyl group, X a valency bond and T an oxygen atom.

4. Compounds according to claim 3, in which $R_1$ and $R_2$ each signify a methyl group, $R_3$ hydrogen, a methoxy, hydroxyl or 1-imidazolyl group and $R_4$ and $R_5$ represent a hydrogen atom.

5. The following compounds which are selected from the group of compounds according to claim 1:
7,7-dimethyl-2-(2-methoxyphenyl)-6,7-dihydro-3H,5H-pyrrolo[2,3-f]benzimidazol-6-one
7,7-dimethyl-2-(4-hydroxyphenyl)-6,7-dihydro-3H,5H-pyrrolo[2,3-f]benzimidazol-6-one
7,7-dimethyl-2-phenyl-6,7-dihydro-3H,5H-pyrrolo[2,3-f]benzimidazol-6-one
7,7-dimethyl-2-[4-(1H-imidazol-1-yl)-phenyl]-6,7-dihydro-3H,5H-pyrrolo-2,3-f]benzimidazol-6-one
2′-(4-methoxyphenyl)-spiro[cyclopentan-1,7′-6′,7′-dihydro-3′H,5′H-pyrrolo[2′,3′-f]benzimidazol-6′-one
7,7-dimethyl-2-(3,4-dichlorophenyl)-6,7-dihydro-3H,5H-pyrrolo[2,3-f]benzimidazol-6-one
7,7-dimethyl-2-(4-trifluoromethylphenyl)-6,7-dihydro-3H,5H-pyrrolo[2,3-f]benzimidazol-6-one
7,7-dimethyl-2-(2-methoxy-4-chlorophenyl)-6,7-dihydro-3H,5H-pyrrolo[2,3-f]benzimidazol-6-one
2′-phenyl-spiro[cylcopentan-1,7′-6′,7′-dihydro-3′H,5′H-pyrrolo[2′,3′-f]benzimidazol]-6-one.

6. Process for the preparation of compounds of the general formula I

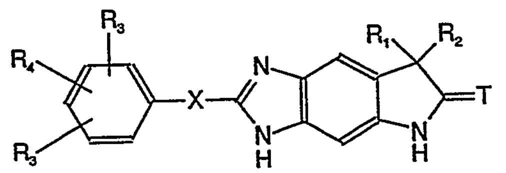
(I),

in which $R_1$ signifies a hydrogen atom, a $C_1$–$C_6$-alkyl, $C_2$–$C_6$-alkenyl or a $C_3$–$C_7$-cycloalkyl group; $R_2$ a hydrogen atom, a $C_1$–$C_6$-alkyl, $C_2$–$C_6$-alkenyl or cyano group, a carbonyl group substituted by a hydroxyl, $C_1$–$C_6$-alkyl, $C_1$–$C_6$-alkoxy, amino,$C_1$–$C_6$- alkylamino, di-$C_1$–$C_6$-alkylamino or hydrazino group or, together with $R_1$, represents a $C_3$–$C_7$-cycloalkylene group or $R_1$ and $R_2$ together form a $C_3$–$C_7$-alkylidene or $C_3$–$C_7$-cycloalkylidene group, $R_3$, $R_4$ and $R_5$ which can be the same or different and each can be hydrogen, an alkanesulphonyloxy, trifluoromethanesulphonyloxy, alkanesulphonylamino, trifluoromethanesulphonylamino, N-alkylalkanesulphonylamino, N-alkyltrifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino group, a sulphonyl group substituted by an amino, alkylamino or dialkylamino group, a morpholino, pyrrolidino, piperidino or hexamethyleneiminosulphonyl group, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, trifluoromethyl, alkoxy, alkenyloxy, alkynyloxy, cyanoalkoxy, carboxyalkoxy, alkoxycarbonylalkoxy, dialkylamino, 1-imidazolyl or cyano group, whereby the alkyl moieties of the above-mentioned substituents contain 1-5 carbon atoms, and X a valency bond, a $C_1$–$C_4$-alky-

lene group or the vinylene group, T represents an oxygen atom; of their tautomers and their physiologically acceptable salts of inorganic and organic acids, characterised in that one either

a) reacts compounds of the general formula II

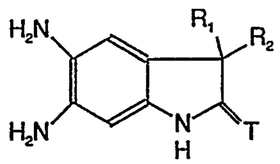

(II),

in which $R_1$, $R_2$ and T have the above-given meaning, with a compound of the general formula III

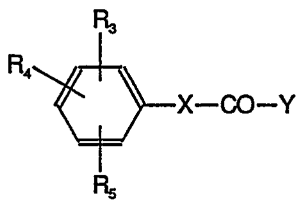

(III),

in which $R_3$, $R_4$, $R_5$ and X possess the given meaning and Y represents either hydrogen or a residue which can easily be split off, and cyclises the compounds obtained to a compound of the formula I or its tautomeric form or

b) acylates compounds of the general formula IV

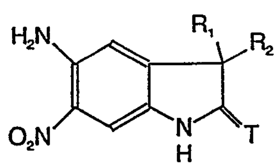

(IV),

or V

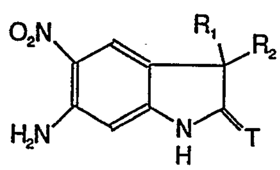

(V),

in which $R_1$, $R_2$ and T have the given meaning, with a compound of the general formula III, in which $R_3$, $R_4$, $R_5$ and X possess the given meaning and Y represents a residue which can easily be split off, according to processes known from the literature to compounds of the general formula:

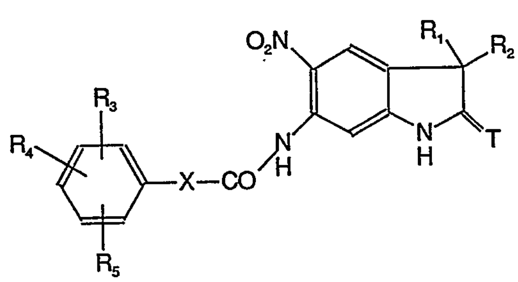

(VI),

or VII

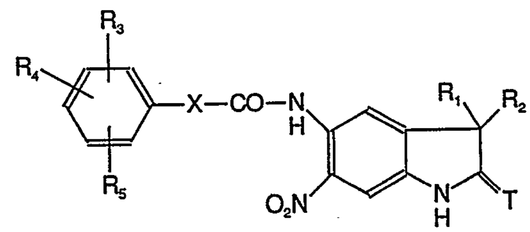

(VII),

in which $R_1$, $R_2$, $R_3$, $R_4$, $R_5$, X and T have the above-given meaning, hydrogenates the nitro group and cyclises the compounds obtained or

c) starting from compounds of the general formula VIII

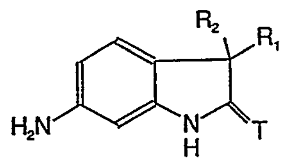

(VIII),

or IX

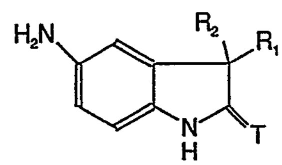

(IX),

in which $R_1$, $R_2$ and T possess the above-given meaning, by reaction with compounds of the general formula III, in which Y represents a residue which can easily be split off, obtains in generally known way, compounds of the general formula:

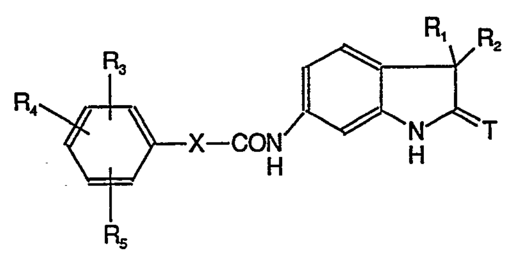

or XI

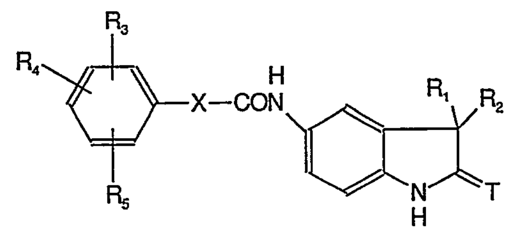

(XI),

in which $R_1$, $R_2$, $R_3$, $R_4$, $R_5$, T and X possess the above-given meaning, and converts in known manner by nitration into compounds of the general formula VI

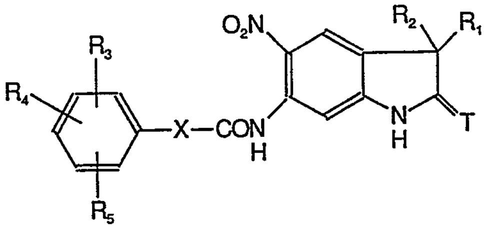

(VI),

or VII

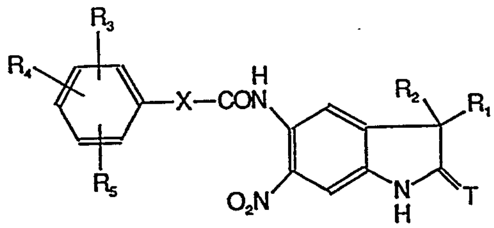

(VII),

in which $R_1$, $R_2$, $R_3$, $R_4$, $R_5$, T and X have the above-given meanings, hydrogenates the nitro group and cyclises the compounds obtained and subsequently, if desired, converts a compound obtained of the formula I into another compound of the formula I or its tautomer and/or converts a compound obtained of the general formula I or its tautomer into a physiologically acceptable acid-addition salt with an inorganic or organic acid.

7. Process according to claim 6 for the preparation of compounds of the general formula I

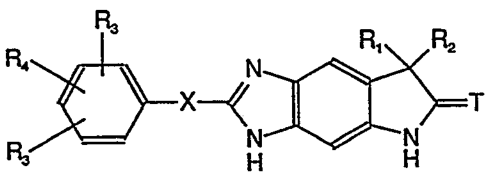

(I),

in which $R_1$ represents a hydrogen atom, the methyl or ethyl group and $R_2$ signifies the methyl, ethyl, isopropyl, cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$, with the C-atom to which they are bound, form a $C_3$-$C_7$-cycloalkyl ring; $R_3$ a hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino or the 1-imidazolyl group, $R_4$ signifies hydrogen, the methyl, methoxy, dimethylamino or chloro group; $R_5$ is hydrogen or the methoxy group, X is a valency bond, an alkylene group with 1 or 2 carbon atoms or the vinylene group; and T stands for an oxygen atom.

8. Medicaments containing a compound according to claim 1 to 5, besides usual carrier and adjuvant materials.

9. Compounds according to claim 1 to 5 for the prophylaxis or combating of heart and circulatory diseases.

10. Use of compounds according to claim 1 to 5 and 6 for the preparation of medicaments for the prophylaxis or combating of heart and circulatory diseases.

**Revendications**

1. Composés de formule générale I

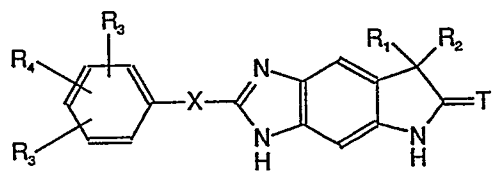

(I),

où

$R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$-, un groupe alcényle en $C_2$-$C_6$ ou un groupe cyano, un groupe carbonyle substitué par un groupe hydroxy, un groupe alkyle en $C_1$-$C_6$, alcoxy en $C_1$-$C_6$, amino, (alkyle en$C_1$-$C_6$)-amino, di(alkyle en $C_1$-$C_6$)amino ou hydrazino, ou, conjointement avec $R_1$, représente un groupe cycloalkylène en $C_3$-$C_7$, ou $R_1$ et $R_2$ forment, conjointement, un groupe alkylidène en $C_3$-$C_7$ ou cycloalkylidène en $C_3$-$C_7$,

$R_3$, $R_4$ et $R_5$ peuvent être identiques ou différents et peuvent représenter chacun un atome d'hydrogène, un groupe alcanesulfonyloxy, trifluorométhanesulfonyloxy, alcanesulfonylamino, trifluorométhanesulfonylamino, N-alkylalcanesulfonylamino, N-alkyltrifluorométhanesulfonylamino, alkylsulfonylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino ou dialkylamino, un groupe morpholino, pyrrolidino, pipéridino ou hexaméthylèneiminosulfonyle, un groupe alkylcarbonylamino, aminocarbonylamino ou alkylaminocarbonylamino, un groupe alkylmercapto, alkylsulfinyle ou alkylsulfonyle, un groupe nitro, halogéno, amino, hydroxy, alkyle, trifluorométhyle, alcoxy, alcényloxy, alcinyloxy, cyanalkyloxy, carboxyalkyloxy, alcoxycarbonylalkyloxy, dialkylamino, 1-imidazolyle ou cyano, les parties alkyle contenant les substituants à 1-5 atomes de carbone mentionnés ci-dessus, et

X peut être une valence libre, un groupe alkylène en $C_1$-$C_4$ ou un groupe vinylène,

T représente un atome d'oxygène,

leurs tautomères et leurs sels physiologiquement compatibles d'acides minéraux et organiques.

2. Composés de formule générale I selon la revendication 1,

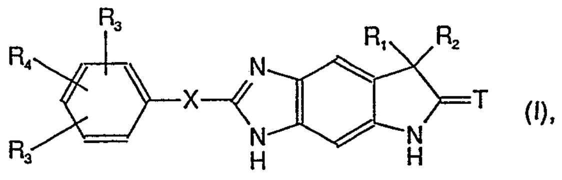 (I),

où

$R_1$ représente un atome d'hydrogène, un groupe méthyle ou éthyle,

et $R_2$ représente un groupe méthyle, éthyle, isopropyle, cyano, acétyle, méthoxycarbonyle, éthoxycarbonyle, aminocarbonyle et hydrazinocarbonyle, $R_1$ et $R_2$ représentent un cycle spirocyclopentyle, lorsque $R_1$ et $R_2$ forment, avec l'atome de C auquel ils sont liés, un cycle cycloalkyle en $C_3$-$C_7$.

$R_3$ représente un atome d'hydrogène, un groupe méthanesulfonyloxy, trifluorométhanesulfonyloxy, méthanesulfonylamino, trifluorométhanesulfonylamino, méthanesulfonyl-méthylamino, trifluorométhanesulfonylméthylamino, méthylsulfenylméthyle, méthylsulfinylméthyle, méthylsulfonylméthyle, aminocabonyle, aminosulfonyle, méthylaminosulfonyle, diméthylaminosulfonyle, acétylamino, méthylmercapto, méthylsulfonyle, hydroxy, méthyle, méthoxy, propargyloxy, cyanméthyloxy, méthoxycarbonylméthyloxy, cyano, chloro, nitro, amino, diméthylamino ou 1-imidazolyle,

$R_4$ représente un atome d'hydrogène, un groupe méthyle, méthoxy, diméthylamino ou chloro,

$R_5$ représente un atome d'hydrogène ou un groupe méthoxy,

X représente une valence libre, un groupe alkylène à 1 ou 2 atomes de carbone ou un groupe vinylène,

T représente un atome d'oxygène, leurs tautomères et leurs sels physiologiquement compatibles d'acides minéraux et organiques.

3. Composés selon la revendication 1 ou 2, dans lesquels $R_1$ et $R_2$ représentent chacun un groupe alkyle en $C_1$-$C_6$, ou $R_1$ et $R_2$ représentent un cycle spirocyclopentyle,

$R_3$, $R_4$ et $R_5$ peuvent être identiques ou différents et peuvent représenter chacun un atome d'hydrogène, un groupe alcoxy en $C_1$-$C_5$, hydroxy ou 1-imidazolyle, X représente une valence libre et T représent un atome d'oxygène.

4. Composés selon la revendication 3, dans lesquels $R_1$ et $R_2$ représentent chacun un groupe méthyle, $R_3$ représente un atome d'hydrogène, un groupe méthoxy, hydroxy ou 1-imidazolyle, et $R_4$ et $R_5$ représentent un atome d'hydrogène.

5. Composés énumérés ci-après, choisis dans le groupe des composés selon la revendication 1:

7,7-diméthyl-2-(2-méthoxy-phényl)-6,7-dihydro-3H,5H-pyrrolo-[2,3-f] benzimidazol-6-one

7,7-diméthyl-2-(4-hydroxy-phényl)-6,7-dihydro-3H,5H-pyrrolo-[2,3-f] benzimidazol-6-one

7,7-diméthyl-2-phényl-6,7-dihydro-3H,5H-pyrrolo-[2,3-f] benzimidazol-6-one

7,7-diméthyl-2-[4(1H-imidazol-1-yl)-phényl]-6,7-dihydro-3H,5H-pyrrolo-[2,3-f] benzimidazol-6-one

2'-(4-méthoxy-phényl)-spiro[cyclopentan-1,7'-6',7'-dihydro-3'H,5'H-pyrrolo-[2,3-f]benzimidazol]-6'-one

7,7-diméthyl-2-(3,4-dichlorophényl)-6,7-dihydro-3H,5H-pyrrolo-[2,3-f] benzimidazol-6-one

7,7-diméthyl-2-(4-trifluorométhyl-phényl)-6,7-dihydro-3H,5H-pyrrolo-[2,3-f] benzimidazol-6-one

7,7-diméthyl-2-(2-méthoxy-4-chloro-phényl)-6,7-dihydro-3H,5H-pyrrolo-[2,3-f] benzimidazol-6-one

2'-phényl-spiro[cyclopentan-1,7'-6',7'-dihydro-3'H,5'H-pyrrolo-[2,3-f]benzimidazol]-6'-one

6. Procédé de préparation de composés de formule générale I

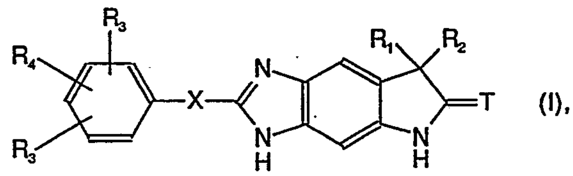 (I),

dans lesquels

$R_1$ représente un atome d'hydrogène, un groupe alkyle en $C_1$-$C_6$, un groupe alcényle en $C_2$-$C_6$ ou un groupe cycloalkyle en $C_3$-$C_7$,

$R_2$ représente un atome d'hydrogène, un groupe alkyle en $C_1$-$C_6$, un groupe alcényle en $C_2$-$C_6$ ou un groupe cyano, un groupe carbonyle substitué par un groupe hydroxy, un groupe alkyle en $C_1$-$C_6$, alcoxy en $C_1$-$C_6$, amino, (alkyle en$C_1$-$C_6$)-amino, di(alkyle en $C_1$-$C_6$)amino ou hydrazino, ou, conjointement avec $R_1$, représente un groupe cycloalkylène en $C_3$-$C_7$, ou $R_1$ et $R_2$ forment, conjointement, un groupe alkylidène en $C_3$-$C_7$ ou cycloalkylidène en $C_3$-$C_7$,

$R_3$, $R_4$ et $R_5$ peuvent être identiques ou différents et peuvent représenter chacun un atome d'hydrogène, un groupe alcanesulfonyloxy, trifluorométhanesulfonyloxy, alcanesulfonylamino, trifluorométhanesulfonylamino, N-alkylalcanesulfonylamino, N-alkyltrifluorométhanesulfonylamino, alkylsulfonylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino ou dialkylamino, un groupe morpholino, pyrrolidino, pipéridino ou hexaméthylèneiminosulfonyle, un groupe alkylcarbonylamino, aminocarbonylamino ou alkylaminocarbonylamino, un groupe alkylmercapto, alkylsulfinyle ou alkylsulfonyle, un groupe nitro, halogéno, amino, hydroxy, alkyle, trifluorométhyle, alcoxy, alcényloxy, alcinyloxy, cyanalkyloxy, carboxyalkyloxy, alcoxycarbonylalkyloxy, dialkylamino, 1-imidazolyle ou cyano, les parties alkyle contenant les substituants 1-5 atomes de carbone mentionnés ci-dessus, et

X peut être une valence libre, un groupe alkylène en $C_1$-$C_4$ ou un groupe vinylène,

T représente un atome d'oxygène,

leurs tautomères et leurs sels physiologiquement compatibles d'acides minéraux et organiques, caractérisé en ce que soit

a) on fait réagir des composés de formule générale II

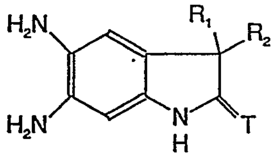

(II),

dans lesquels
$R_1$, $R_2$ et T ont les significations mentionnées, avec un composé de formule générale III

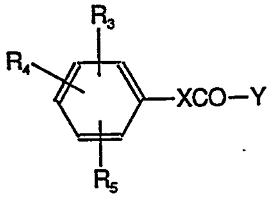

(III),

où
$R_3$, $R_4$, $R_5$ et X ont les significations mentionnées et Y représente soit un atome d'hydrogène soit un groupe facilement séparable, et on cyclise les composés obtenus en un composé de formule I ou en ses tautomères, soit
b) on fait réagir des composés de formule générale IV

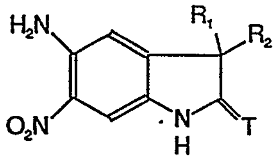

(IV),

où V

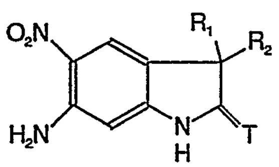

(V),

où
$R_1$, $R_2$ et T ont les significations mentionnées, avec un composé de formule générale III
où
$R_3$, $R_4$, $R_5$ et X ont les significations mentionnées et Y représente un groupe facilement séparable, suivant des procédés décrits dans la littérature, de manière à les transformer en composés de formule générale VI,

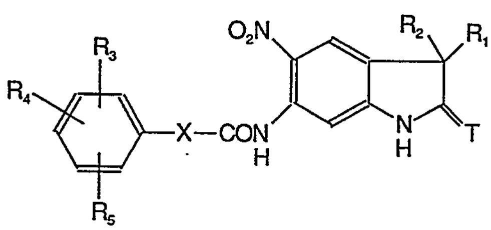

(VI),

où VII

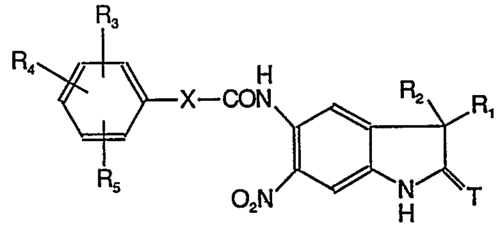

(VII),

dans lesquels
$R_1$, $R_2$, $R_3$, $R_4$, $R_5$, T et X ont les significations mentionnées plus haut, on acyle, hydrogène le groupe nitro et cyclise les composés obtenus, soit
c) en partant des composés de formule générale VIII

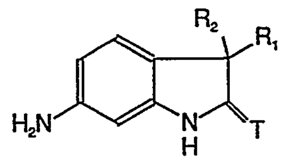

(VIII),

où IX

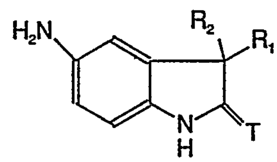

(IX),

dans lesquels $R_1$, $R_2$ et T ont les significations mentionnées plus haut, par réaction avec des composés de formule générale III, dans lesquels Y représente un groupe facilement séparable, de manière connue en soi, on transforme les composés de formule X

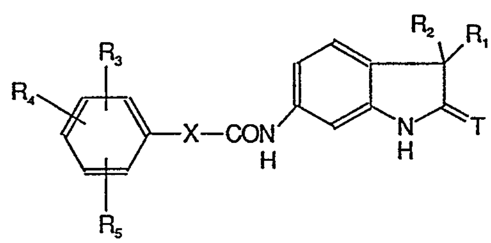

(X),

où XI

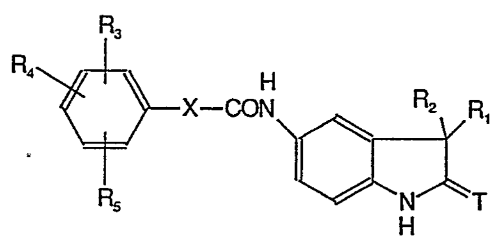

(XI),

où $R_1$, $R_2$, $R_3$, $R_4$, $R_5$, T et X ont les significations mentionnées plus haut, et on les transforme, de manière connue en soi, par nitration, en composés de formule générale VI

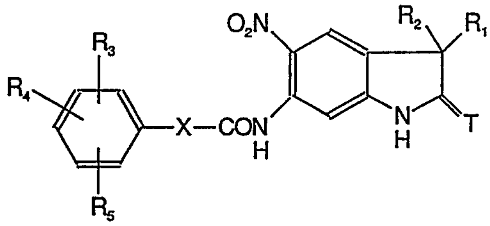

(VI),

où VII

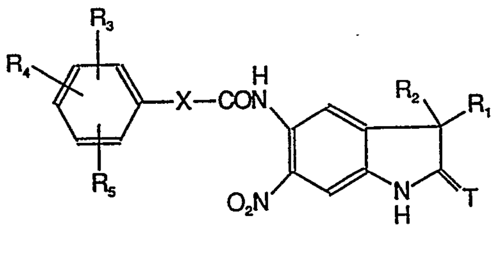

(VII),

où $R_1$, $R_2$, $R_3$, $R_4$, $R_5$, T et X ont les significations mentionnées plus haut, on hydrogène le groupe nitro et on cyclise les composés obtenus, et ensuite, on transforme éventuellement un composé de formule I obtenu selon l'invention en un autre composé de formule I ou en son tautomère, et/ou un composé de formule générale I obtenu et ses tautomères, en un sel d'addition d'acide physiologiquement compatible, avec un acide minéral ou organique.

7. Procédé selon la revendication 6, pour la préparation de composés de formule générale I

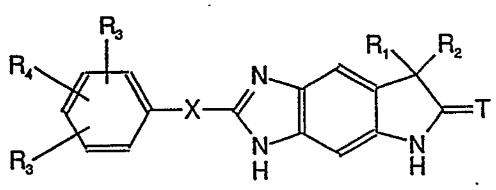

(I),

dans lesquels

$R_1$ représente un atome d'hydrogène, un groupe méthyle ou éthyle,

et $R_2$ représente un groupe méthyle, éthyle, isopropyle, cyano, acétyle, méthoxycarbonyle, éthoxycarbonyle, aminocarbonyle et hydrazinocarbonyle, $R_1$ et $R_2$ représentent un cycle spirocyclopentyle, lorsque $R_1$ et $R_2$ forment, avec l'atome de C auquel ils sont liés, un cycle cycloalkyle en $C_3$-$C_7$,

$R_3$ représente un atome d'hydrogène, un groupe méthanesulfonyloxy, trifluorométhanesulfonyloxy, méthanesulfonylamino, trifluorométhanesulfonylamino, méthanesulfonyl-méthylamino, trifluorométhanesulfonylméthylamino, méthylsulfonylméthyle, méthylsulfinylméthyle, méthylsulfonylméthyle, aminocarbonyle, aminosulfonyle, méthylaminosulfonyle, diméthylaminosulfonyle, acétylamino, méthylmercapto, méthylsulfonyle, hydroxy, méthyle, méthoxy, propargyloxy, cyanméthyloxy, méthoxycarbonylméthyloxy, cyano, chloro, nitro, amino, diméthylamino ou 1-imidazolyle,

$R_4$ représente un atome d'hydrogène, un groupe méthyle, méthoxy, diméthylamino ou chloro,

$R_5$ représente un atome d'hydrogène ou un groupe méthoxy,

X représente une valence libre, un groupe alkylène à 1 ou 2 atomes de carbone ou un groupe vinylène, et

T représente un atome d'oxygène.

8. Produit pharmaceutique, contenant un composé selon les revendications 1 à 5, et des substances de support et auxiliaires usuelles.

9. Composés selon les revendications 1 à 5, pour le traitement préventif ou curatif des affections cardiovasculaires.

10. Utilisation des composés selon les revendications 1 à 5 pour le traitement préventif ou curatif des affections cardiovasculaires.